(19) Europäisches Patentamt
European Patent Office
Office européen des brevets

(11) Publication number : **0 391 591 B1**

(12) # EUROPEAN PATENT SPECIFICATION

(45) Date of publication of patent specification : **15.06.94 Bulletin 94/24**

(51) Int. Cl.$^5$ : **C07D 239/26,** C09K 19/34

(21) Application number : **90303268.8**

(22) Date of filing : **28.03.90**

(54) **Pyrimidine derivative and liquid crystal composition incorporating the same.**

(30) Priority : **06.04.89 JP 87428/89**
**06.04.89 JP 87429/89**
**11.09.89 JP 234896/89**
**12.10.89 JP 265665/89**
**20.11.89 JP 301451/89**

(43) Date of publication of application : **10.10.90 Bulletin 90/41**

(45) Publication of the grant of the patent : **15.06.94 Bulletin 94/24**

(84) Designated Contracting States : **CH DE GB LI**

(56) References cited : **WO-A-86/04081**

(73) Proprietor : **SEIKO EPSON CORPORATION**
**4-1, Nishishinjuku 2-chome**
**Shinjuku-ku Tokyo (JP)**

(72) Inventor : **Obikawa, Tsuyoshi, c/o Seiko Epson Corporation**
**3-5 Owa 3-chome**
**Suwa-shi, Nagano-ken (JP)**
Inventor : **Yamada, Shuhei, c/o Seiko Epson Corporation**
**3-5 Owa 3-chome**
**Suwa-shi, Nagano-ken (JP)**
Inventor : **Ikukawa, Shuji, c/o Seiko Epson Corporation**
**3-5 Owa 3-chome**
**Suwa-shi, Nagano-ken (JP)**

(74) Representative : **Miller, Joseph et al**
**J. MILLER & CO.**
**34 Bedford Row,**
**Holborn**
**London WC1R 4JH (GB)**

**EP 0 391 591 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to pyrimidine derivatives and to compositions incorporating the same.

Liquid crystal display devices utilising the electro-optical effect of nematic liquid crystal compounds have many applications. Liquid crystal display devices may be of the dynamic scattering type, guest-host type, twisted nematic (TN) type, super twisted nematic (STN) type, etc. Further, liquid crystal display devices may employ, for example, a static driving system, a time divisional (dynamic) driving system, an active matrix driving system or a two frequency driving system.

Liquid crystal display devices, in particular, those of the TN type and the STN type have excellent features as compared with display devices of the light emission type such as, for example, LED, EL and CRT, namely:-

(1) Reduced size, thickness and weight.

(2) Low driving voltage and less power consumption.

(3) Good compatibility with LSI and simple driving circuitry.

(4) Since liquid crystal display devices are of the light receiving type, their display is easy to see even under direct sunlight and give less fatigue to the eyes even for long periods of use.

In view of the above, liquid crystal display devices mainly of the TN type have generally been used for watches, pocket electronic calculators, audio equipment, game devices, car dashboards, cameras, telephone sets and various instruments, and it is expected that their use will be further extended.

While there has been an attempt to increase the display capacity of liquid crystal display devices, it has been found that the number of scanning lines is about 200 at the most using the time divisional driving system which is the predominant system at present. STN type liquid crystal display devices are now in practical use because they are capable of increasing the number of scanning lines. Further, TN type liquid crystal display devices using the active matrix driving system can provide the possibility of considerably increasing the number of scanning lines and have now also been put to practical use. STN type liquid crystal display devices are used for personal computers and word processors at present and TN type liquid crystal display devices using the active matrix driving system are used mainly in liquid crystal colour television sets having attracted attention as a substitute for CRTs.

Various properties are required for liquid crystal compositions used in TN type or STN type liquid crystal display devices and the following requirements are considered to be fundamental and indispensable:-

(1) Free from colouration and having optical, thermal, electrical and chemical stability.

(2) Wide practical temperature range.

(3) Low driving voltage, etc.

Although there are many liquid crystal compounds capable of satisfying requirement (1), individual compounds capable of satisfying both requirements (2) and (3) are, as yet, unknown. In view of the above, liquid crystal compositions prepared by admixing a plurality of nematic liquid crystal compounds or analogous compounds are currently used to satisfy the above requirements.

The lower limit for the practical temperature range (requirement (2)) can be reduced by setting the ratio between each of the ingredients in a liquid crystal composition to that of an eutectic mixture. The upper limit can be increased by including in the liquid crystal composition a so called high temperature liquid crystal compound having a high N-I point. The driving voltage (requirement (3)) can be reduced by reducing the threshold voltage (Vth) for voltage-luminance characteristics. In the case of a TN type liquid crystal display device, Vth is represented by the following formula:

$$Vth = \frac{\pi}{d}\sqrt{\frac{K_{11} + (k_{33} - 2K_{22})/4}{\varepsilon_0 \Delta \varepsilon}}$$

where

d : thickness of liquid crystal layer,

$\varepsilon_0$ : dielectric constant in vacuum,

$K_{11}$, $K_{22}$ and $K_{33}$ : elastic constant for spray, twist and bend, respectively.

Thus it is possible to lower Vth by adding a compound having a small elastic constant and large $\Delta\varepsilon$.

Liquid crystal compounds used so far for raising the upper limit of the practical temperature range, for example:

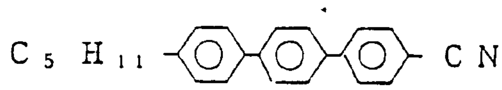

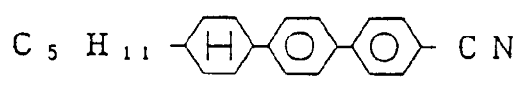

have relatively low N-I points at 239°C and 219°C, respectively, and cannot be added to a liquid crystal composition in great amounts because of their poor compatibility with the other liquid crystal compounds. Accordingly, they do not increase the upper limit sufficiently. Further, although the above two compounds have large $\Delta\varepsilon$, they only reduce Vth by a relatively small amount because of their great elastic constant. Consequently it cannot be said that they are particularly satisfactory compounds as far as reduction of Vth is concerned.

The present invention seeks to provide a nematic liquid crystal compound having an extremely high N-I point and great $\Delta\varepsilon$ and a liquid crystal composition having a wide practical temperature range and a low driving voltage by incorporating a liquid crystal compound according to the present invention in a conventional liquid crystal composition.

According to one aspect of the present invention there is provided a pyrimidine derivative represented by the general formula:

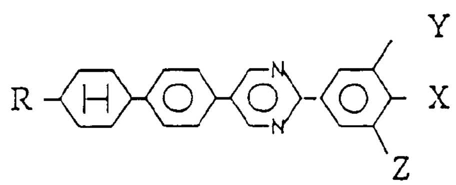

(1)

where R represents a straight chain alkyl group with 1 to 10 carbon atoms, X represents F or CN, Y and Z represent, respectively, H or F, and the cyclohexane ring has a trans arrangement.

According to another aspect of the present invention there is provided a liquid crystal composition comprising at least one pyrimidine derivative according to the present invention.

The pyrimidine derivative according to the present invention may be represented by one of the following general formulae:

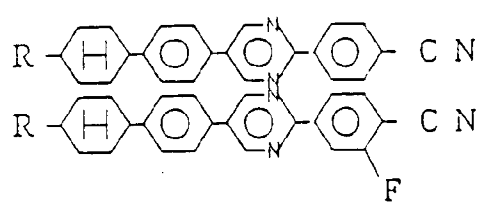

(1 - a)

(1 - b)

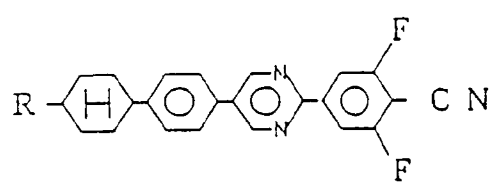

(1 - c)

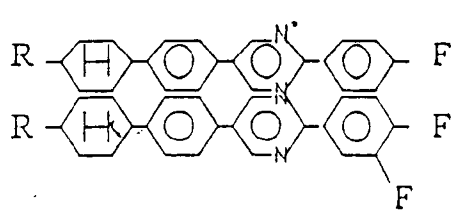

(1 - d)

(1 - e)

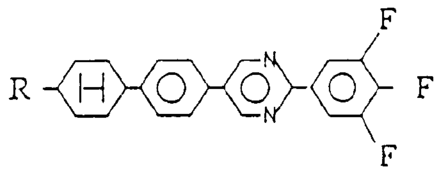 (1 - f)

Pyrimidine derivatives according to the present invention have extremely high N-I points as shown in Table 1 below. Further, pyrimidine derivatives, in particular, the compounds represented by the general formulae (1-b), (1-c) and 1-f) have very large dielectric anisotropy.

Accordingly, it is possible to obtain a liquid crystal composition having a high upper limit for the practical temperature range and a low driving voltage (Vth) by adding a pyrimidine derivative according to the present invention in a small amount to a conventional nematic liquid crystal composition.

Table 1

| Compound | (N-I) point (℃) |
|---|---|
| 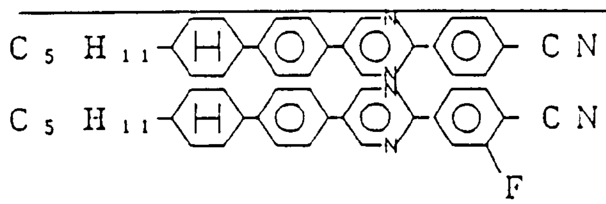 | >360 |
| | 346.5 |
| 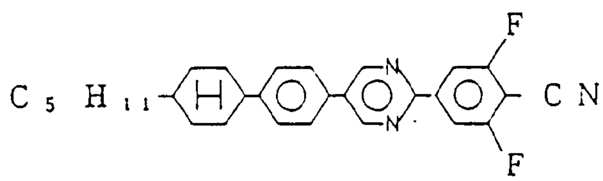 | 307.7 |
| 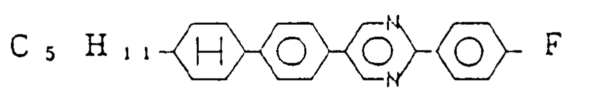 | 316.9 |
| 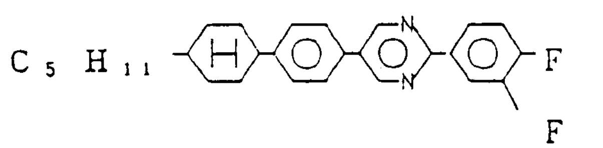 | 287.7 |

There are no particular restrictions as to the liquid crystal compounds that can be admixed with pryimidine derivatives according to the present invention, and a liquid crystal composition having a wide practical temperature range and a low driving voltage can be obtained by admixing, for example, a pyrimidine derivative according to the present invention with one or more of the following types of liquid crystal compound:

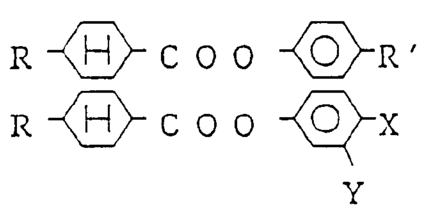

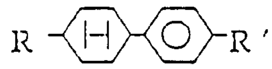

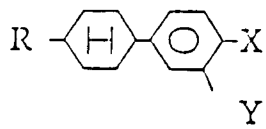

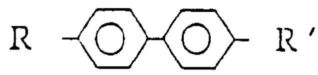

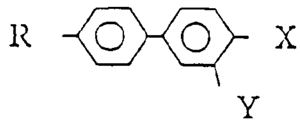

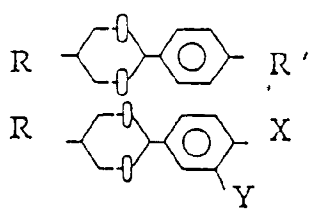

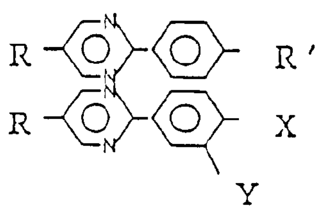

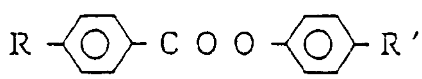

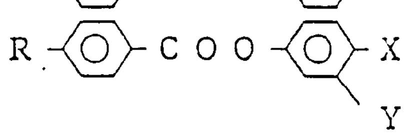

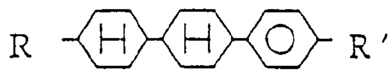

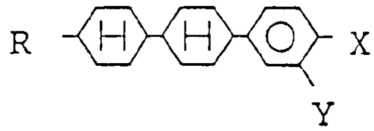

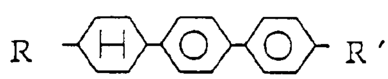

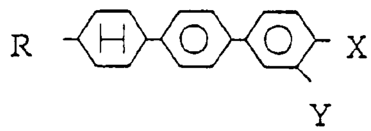

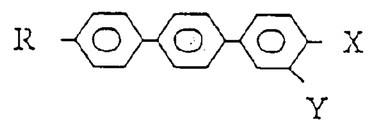

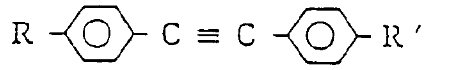

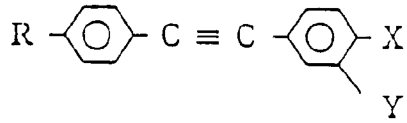

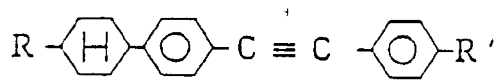

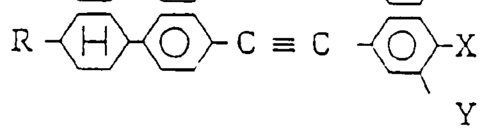

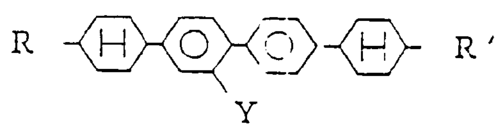

where R and R′ represent, respectively, straight chain alkyl or alkoxy group, X represents F or CN, Y represents H, F or C1.

Pyrimidine derivatives according to the present invention have a good compatibility with conventional liquid crystal compositions and can be mixed therewith within a range from 1 to 30% by weight of the conventional liquid crystal composition. A preferred range is from 3 to 20% by weight when considering the possibility of crystallisation at low temperature.

Further, when a pyrimidine derivative according to the present invention is admixed with a conventional liquid crystal composition, a wider practical temperature range and lower driving voltage can, of course, be attained by mixing a single pyrimidine derivative, but the effect is enhanced by using a plurality of pyrimidine derivatives together. In particular, if it is intended to add a pyrimidine derivative according to the present invention in an amount which is more than 10% by weight, it is desirable to use a plurality of pyrimidine derivatives in admixture since the use of a single pyrimidine derivative may possibly cause problems from the view point of compatibility.

In the present invention, a pyrimidine derivative represented by the general formula:

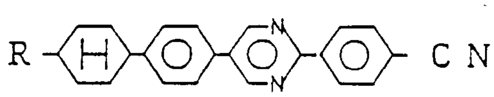

(1 − a)

where R represents a straight chain alkyl group with 1 to 10 carbon atoms and the cyclohexane ring has a trans arrangement can be prepared in accordance with Reaction Schemes 1, 2 and 3 shown below. In Reaction Scheme 1, starting from 4-(trans-4′-alkyl-cyclohexyl) benzoic acid, 1-dimethylamino-3-dimethylimino-2-(4′-(trans-4′-alkylcyclohexyl)phenyl)-1-propene perchlorate (compound (8)) can be obtained by six reaction steps. In Reaction Scheme 2, starting from 4-bromobenzoic acid, 4-bromobenzamidine hydrogen chloride (compound 14)) can be obtained by five reaction steps. In Reaction Scheme 3, 2-(4′-cyanophenyl)-5-(5′-(trans-4″-alkylcyclohexyl) phenyl)pyrimidine (1-a) can be obtained from compound (8) and compound (14) in two reaction steps.

The outline of the process illustrated in Reaction Scheme 1 is as follows:

## Reaction Scheme 1

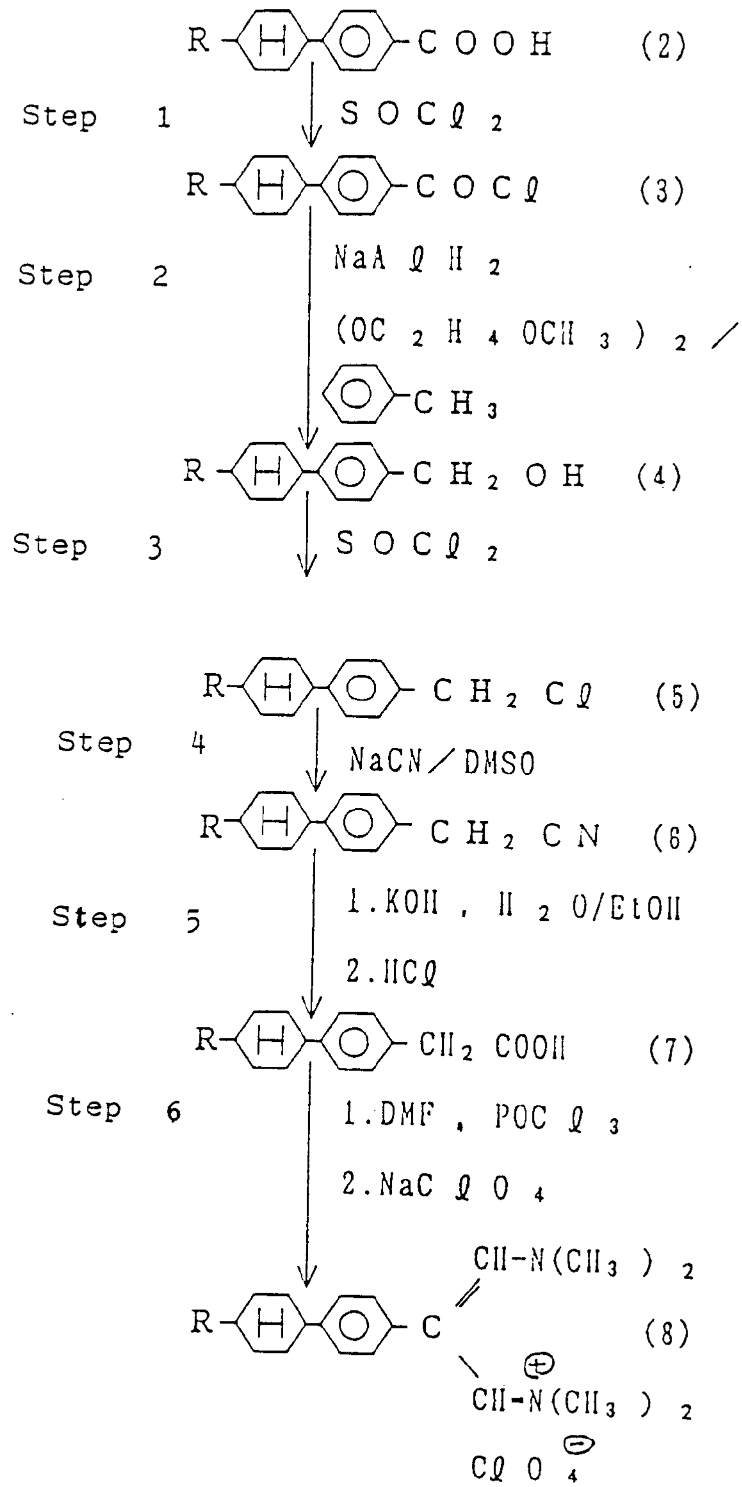


where R represents a straight chain alkyl group with 1 to 10 carbon atoms and the cyclohexane ring has a trans arrangement.

**Step 1**

4-(trans-4′-alkylcyclohexyl) benzoic acid (compound (2)) is chlorinated using thionyl chloride, to obtain 4-(trans-4′-alkylcyclohexyl) benzoic acid chloride (compound (3)).

**Step 2**

Compound (3) is reduced in toluene with hydrogenated bis (methoxyethoxy) aluminium sodium, to obtain 4-(trans-4′-alkylcyclohexyl) benzyl alcohol (compound (4)).

**Step 3**

Compound (4) is chlorinated using thionyl chloride, to obtain 4-(trans-4′-alkylcyclohexyl) benzyl chloride (compound (5)).

**Step 4**

Compound (5) is cyanated using sodium cyanate in dimethyl sulphoxide (DMSO), to obtain 4-(trans-4′-alkylcyclohexyl) phenyl acetonitrile (compound (6)).

**Step 5**

Compound (6) is hydrolyzed in ethanol using water and potassium hydroxide and then neutralised with hydrogen chloride, to obtain 4-(trans-4′-alkylcyclohexyl) phenyl acetic acid (compound (7)).

**Step 6**

Compound (7) is brought into reaction with Vilsmeier reagent prepared from N,N-dimethylformamide (DMF) and phosphorus oxychloride and then formed into a perchlorate using an aqueous solution of sodium perchlorate, to obtain 1-dimethylamino-3-dimethylimino-2-(4′-(trans-4′-alkylcyclohexyl) phenyl)-1-propene perchlorate (compound (8)).

The outline of the process illustrated in Reaction Scheme 2 is as follows:-

Reaction Scheme 2

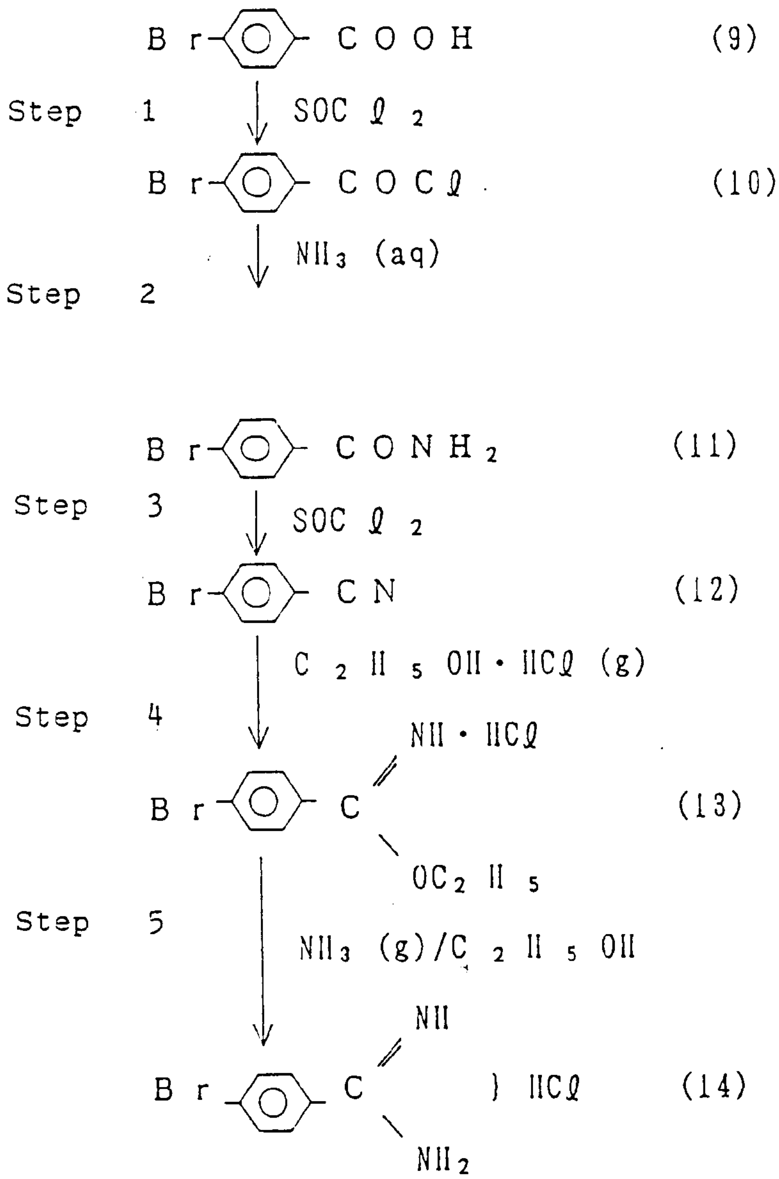


**Step 1**

4-bromobenzoic acid (compound (9)) is chlorinated using thionyl chloride, to obtain 4-bromo-benzoic acid (compound (10)).

**Step 2**

Compound (10) is brought into reaction with aqueous ammonia, to obtain 4-bromobenzoic acid amide (compound (11)).

**Step 3**

Compound (11) is dehydrated using thionyl chloride to obtain 4-bromobenzonitrile (compound (12)).

**Step 4**

Compound (12) is brought into reaction with gaseous hydrogen chloride in anhydrous ethanol, to obtain 4-bromobenzimidate hydrogen chloride (compound (13)).

**Step 5**

Compound (13) is reacted in ethanol using gaseous ammonia, to obtain 4-bromobenzamidine hydrogen chloride (compound (14)).

Then in accordance with Reaction Scheme 3:

Reaction Scheme 3

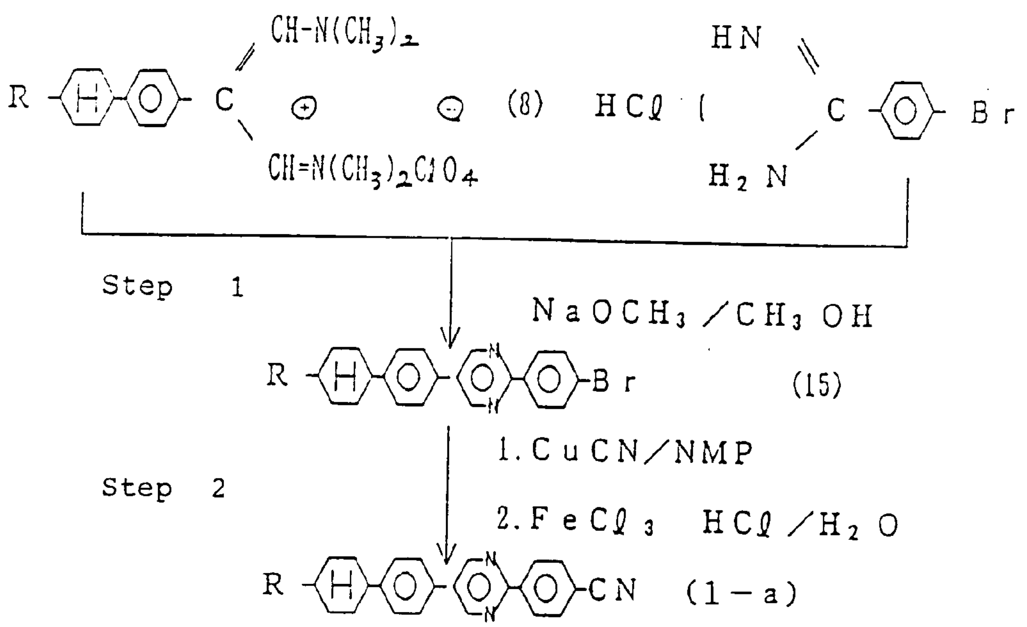


(14)

where R represents a straight chain alkyl group with 1 to 10 carbon atoms and the cyclohexane ring has a trans arrangement.

**Step 1**

Compound (8) and compound (14) are brought into reaction using sodium methoxide in anhydrous methanol to obtain 2-(4′-bromophenyl)-5-(4′-trans-4″-alkylcyclohexyl)phenyl)pyrimidine (compound (15)).

**Step 2**

Compound (15) is cyanated in a mixture of N-methyl-2-pyrrolidinone (NMP) and copper (I) cyanide and the resultant complex is decomposed with hydrochloric acid solution of ferric chloride III to obtain 2-(4′-cyanophenyl)-5-(4′-(trans-4″-alkylcyclohexyl)phenyl) pyrimidine according to the present invention.

In the present invention, a pyrimidine derivative represented by the general formula:

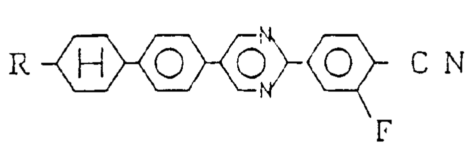

(1 - b)

where R represents a straight chain alkyl group with 1 to 10 carbon atoms and the cyclohexane ring has a trans arrangement can be produced in accordance with Reaction Schemes 1, 4 and 5.

In reaction Scheme 4, starting from 4-bromo-2-fluoroaniline (compound (16)), 4-bromo-3-fluorobenzamidine hydrogen chloride (compound (20)) is obtained by four reaction steps. In Reaction Scheme 5, from compound (8) and compound (20), 2-(4′-cyano-3′-fluorophenyl)-5-(4′-(trans-4″-alkylcyclohexyl)phenyl) pyrimidine (1-b) is obtained by two reaction steps.

The outline for the process illustrated in Reaction Scheme 4 is as follows:

Reaction Scheme 4

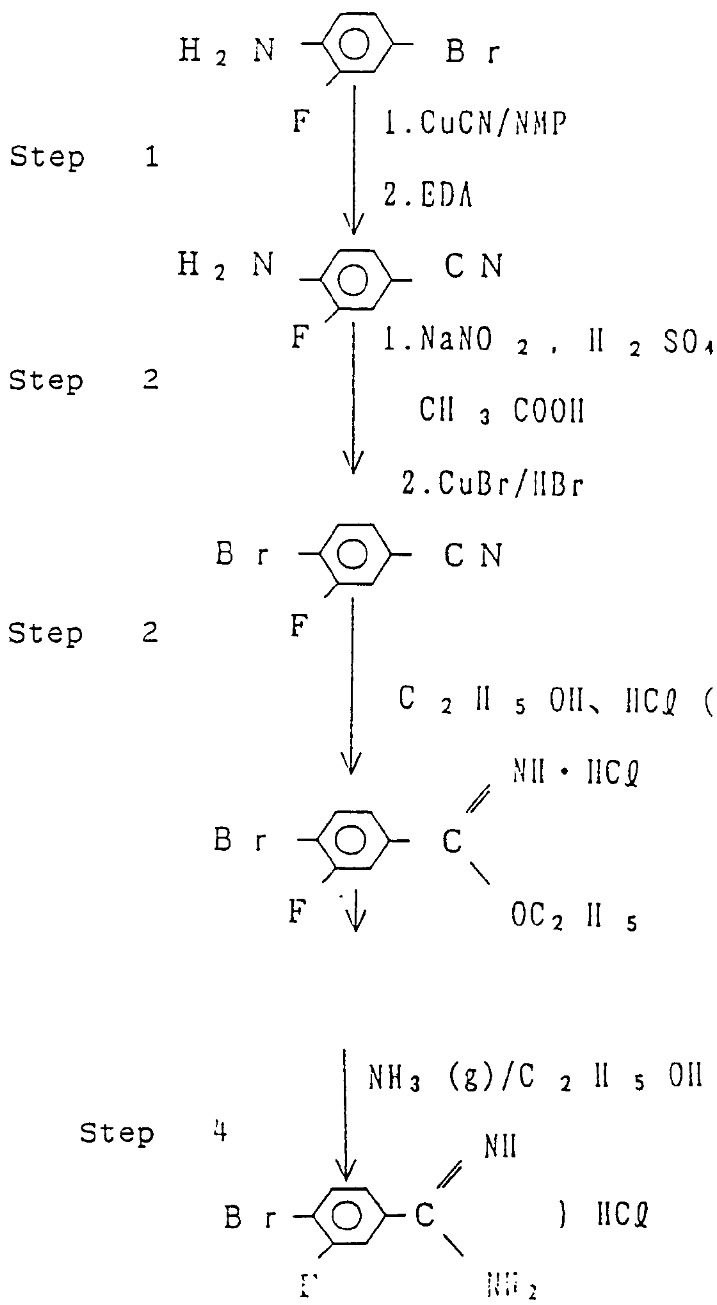


(16)

(17)

(18)

(19)

(20)

**Step 1**

Commercially available 4-bromo-2-fluoroaniline (compound (16)) is cyanated by using copper (I) cyanide in NMP and the resultant copper complex is decomposed with ethylene diamine (EDA), to obtain 4-amino-3-fluorobenzonitrile (compound (17)).

**Step 2**

Compound (17) is converted into its diazo form by using nitrosyl hydrogen sulphate ($HSO_4$-$ONO_2$) prepared from sodium nitrite and concentrated sulphuric acid in glacial acetic acid and then brominated using copper (I) bromide in hydrobromic acid, to obtain 4-bromo-3-fluorobenzonitrile (compound (18)).

**Step 3**

Compound (18) is brought into reaction with gaseous hydrogen chloride in anhydrous ethanol, to obtain 4-bromo-3-fluorobenzimidate hydrogen chloride (compound (19)).

**Step 4**

Compound (19) is brought into reaction with gaseous ammonia in ethanol to obtain 4-bromo-3-fluorobenzamidine hydrogen chloride (compound (20)).

Then in accordance with Reaction Scheme 5:

Reaction Scheme 5

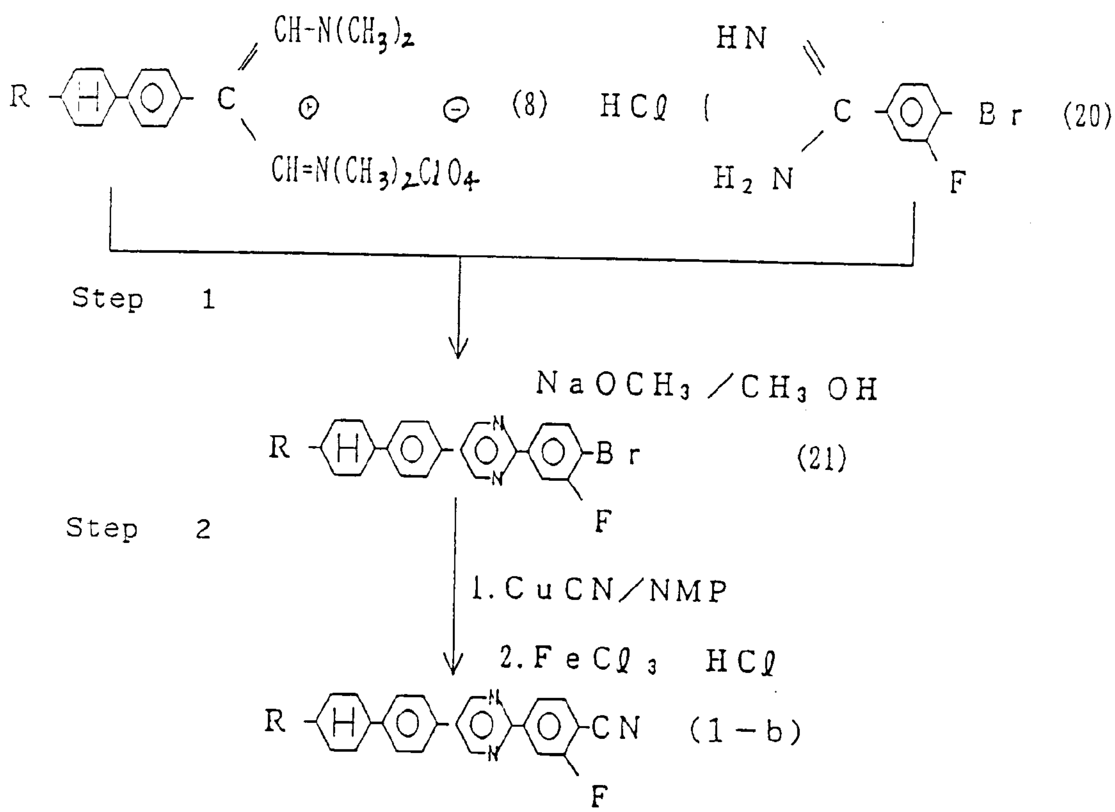


where R represent a straight chain alkyl group with 1 to 10 carbon atoms and the cyclohexane ring has a trans arrangement.

**Step 1**

Compound (8) and compound (20) are reacted by using sodium methoxide in anhydrous methanol, to obtain 2- (4′-bromo-3′-fluorophenyl)-5-(4′-(trans-4″-alkylcyclohexyl)phenyl) pyrimidine (compound (21)).

**Step 2**

Compound (21) was cyanated by using copper (I) cyanide in NMP and the resultant copper complex is decomposed in a hydrochloric acid solution of ferric chloride III, to obtain 2-(4′-cyano-3′-fluorophenyl)-5-(4′-(trans-4″-alkylcyclohexyl)phenyl) pyrimidine (1-b) according to the present invention.

In the present invention, a pyrimidine derivative represented by the general formula:

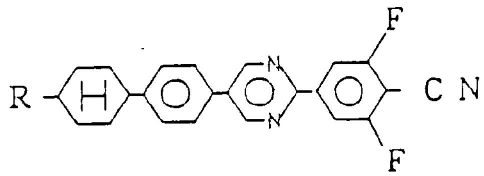

(1 − c)

where R represents a straight chain alkyl group with 1 to 10 carbon atoms and the cyclohexane ring has a trans arrangement, can be produced in accordance with Reaction Schemes 1, 6 and 7. In Reaction Scheme 6, starting from 2,6-difluoroaniline, 4-bromo-3,5-difluorobenzamidine hydrogen chloride (compound (27)) is obtained by five reaction steps. In Reaction Scheme 7, starting from compound (8) and compound (27), 2-(4′-cyano-3′,5′-difluorophenyl)-5-(4′-(trans-4″-alkylcyclohexyl)phenyl) pyrimidine (1-c) is obtained by two reaction steps. The outline of the process illustrated in Reaction Scheme 6 is as follows:-

Reaction Scheme 6

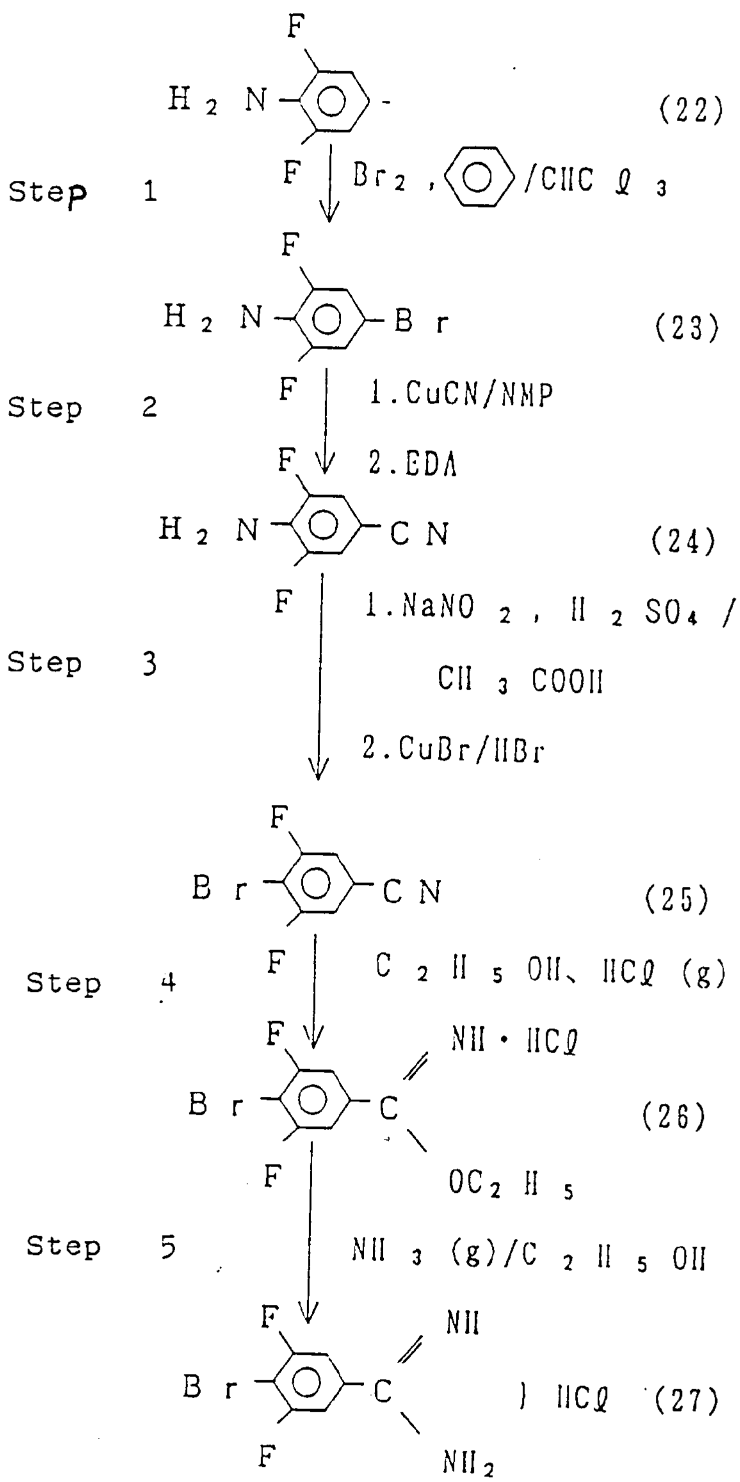


**Step 1**

Commercial available 2,6-difluoroaniline (compound (22)) is brominated in chloroform using bromine in the presence of pyrimidine to obtain 4-bromo-2,6-difluoroaniline (compound (23)).

**Step 2**

Compound (23) is cyanated in NMP using copper (I) cyanide and the resultant copper complex is decomposed with EDA to obtain 4-amino-3,5-difluorobenzonitrile (compound (24)).

**Step 3**

Compound (24) is converted into its diazo form by using nitrosyl hydrogen sulphate prepared from $NaNO_2$ and concentrated sulphuric acid in glacial acetic acid and then brominated using copper (I) bromide in hydrobromic acid, to obtain 4-bromo-3,5-difluorobenzonitrile (compound (25)).

**Step 4**

Compound (25) is reacted using gaseous hydrogen chloride in anhydrous ethanol, to obtain 4-bromo-3,5-difluorobenzimidate hydrogen chloride (compound (26)).

**Step 5**

Compound (26) is reacted in ethanol with gaseous ammonia, to obtain 4-bromo-3,5-difluorobenzamidine hydrogen chloride (compound (27)).

Then in accordance with Reaction Scheme 7:

Reaction Scheme 7

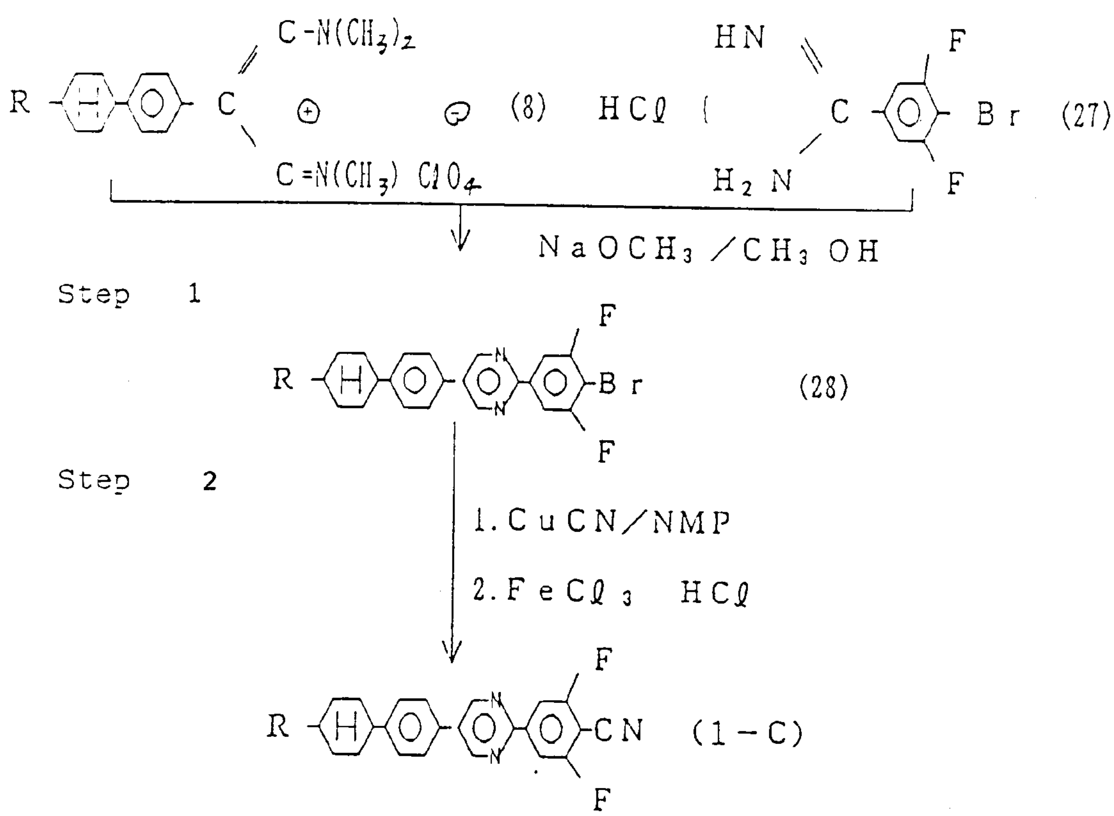


where R represents a straight chain alkyl group with 1 to 10 carbon atoms and the cyclohexane ring has a trans arrangement.

**Step 1**

Compound (8) and compound (27) are reacted by using sodium methoxide in anyhdrous methanol, to obtain 2-(4'-bromo-3',5'-difluorophenyl)-5-(4'-(trans-4''-alkylcyclohexyl)phenyl) pyrimidine (compound (28)).

**Step 2**

Compound (28) is cyanated by using copper (I) cyanide in NMP and the resultant copper complex is decomposed in a hydrochloric acid solution of ferric chloride III, to obtain 2-(4'-cyano-3',5'-difluorophenyl)-5-(4'-(trans-4''-alkylcyclohexyl)phenyl) pyrimidine (1-c) according to the present invention.

In the present invention, a pyrimidine derivative represented by the general formula:

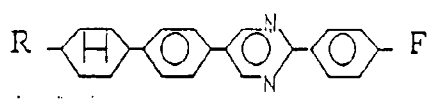

( 1 - d )

where R represents a straight chain alkyl group with 1 to 10 carbon atoms and the cyclohexane ring has a trans arrangement, can be produced in accordance with Reactions Schemes 1, 8 and 9. In Reaction Scheme 8, starting from 4-fluorobenzonitrile, 4-fluorobenzamidine hydrogen chloride (compound (31)) is obtained by two reaction steps. In Reaction Scheme 9, starting from compound (8) and compound (31), 2-(4'-fluorophenyl)-5-(4'-(trans-4''-alkylcyclohexyl)phenyl) pyrimidine (1-d) is obtained by one reaction step.

The outline of the process illustrated in Reaction Scheme 8 is as follows:

Reaction Scheme 8

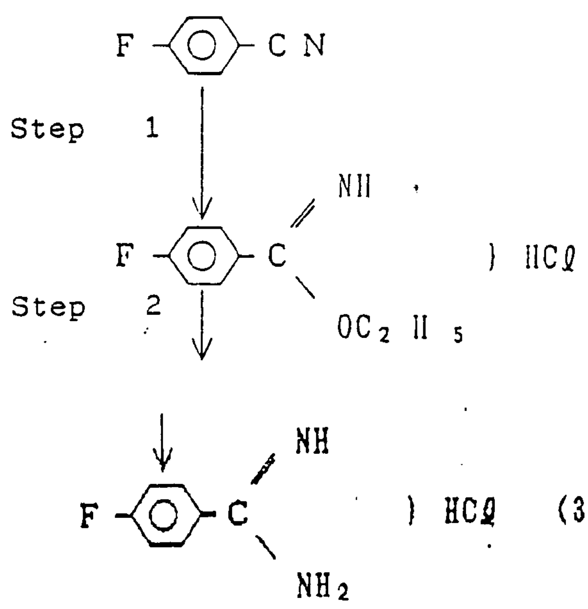


(29)

(30)

(31)

**Step 1**

Commercially available 4-fluorobenzonitrile (compound (29)) is reacted in anhydrous ethanol using gaseous hydrogen chloride, to obtain 4-fluorobenzimidate hydrogen chloride (compound (30)).

**Step 2**

Compound (30) is brought into reaction with gaseous ammonia, to obtain 4-fluorobenzamidine hydrogen chloride (compound (31)).

Then in accordance with Reaction Scheme 9:

Reaction Scheme 9

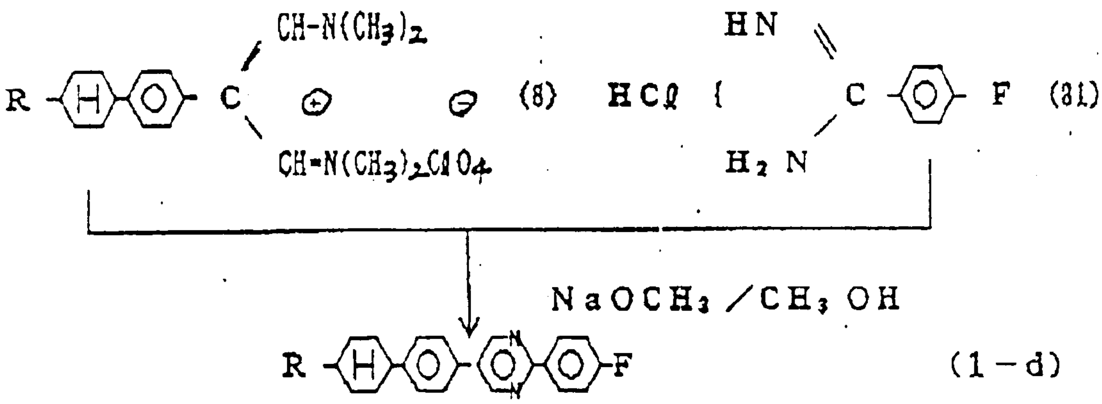


where R represents a straight chain alkyl group with 1 to 10 carbon atoms and the cyclohexane ring has a trans arrangement.

Compound (8) and compound (31) were reacted in anhydrous methanol by using sodium methoxide, to obtain compound 2-(4′-fluorophenyl)-5-(4′-(trans-4″-alkylcyclohexyl)phenyl)pyrimidine (1-d) according to the present invention.

In the present invention, a pyrimidine derivative represented by the general formula:

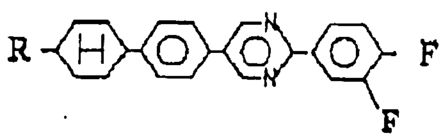
(1 - e)

where R represents a straight chain alkyl group with 1 to 10 carbon atoms and the cyclohexane ring has a trans arrangement, is prepared in accordance with Reaction Schemes 1, 10 and 11. In Reaction Scheme 10, starting from 3,4-difluorobenzonitrile, 3,4-difluorobenzamidine hydrogen chloride (compound (34)) is obtained by two reaction steps. In Reaction Scheme 11, starting from compound (8) and compound (34), 2-(3′,4′-difluorophenyl)-5-(4′-(trans-4″-alkylcyclohexyl)phenyl) pyrimidine (1-e) according to the invention is obtained.

The outline of the process illustrated in Reaction Scheme 10 is as follows:

Reaction Scheme 10

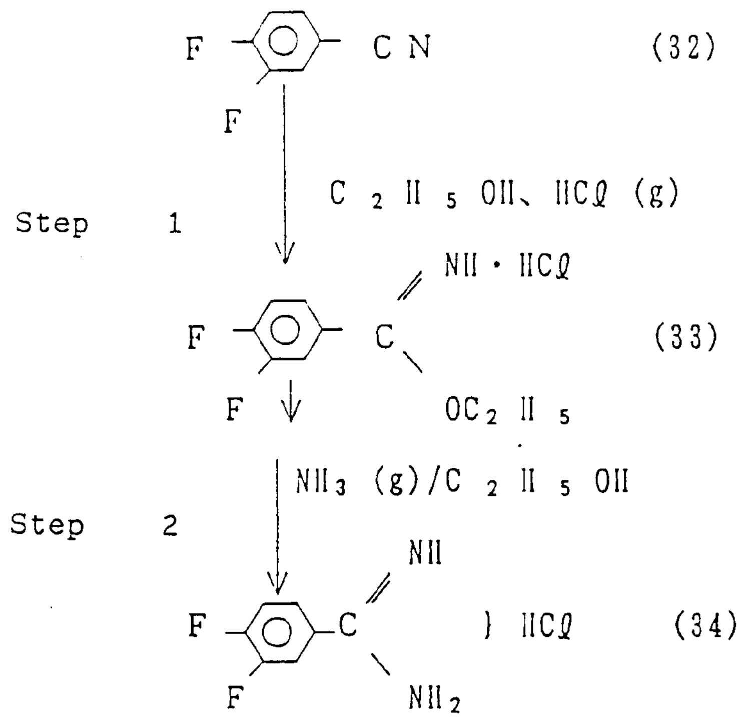


**Step 1**

Commercially available 3,4-difluorobenzonitrile (compound (32)) is reacted using gaseous hydrogen chloride in anhydrous ethanol, to obtain 3,4-difluorobenzimidate hydrogen chloride (compound (33)).

**Step 2**

Compound (33) is brought into reaction with gaseous ammonia in ethanol, to obtain 3,4-difluorobenzamidine hydrogen chloride (compound (34)).

Then in accordance with Reaction Scheme 11:

Reaction Scheme 11

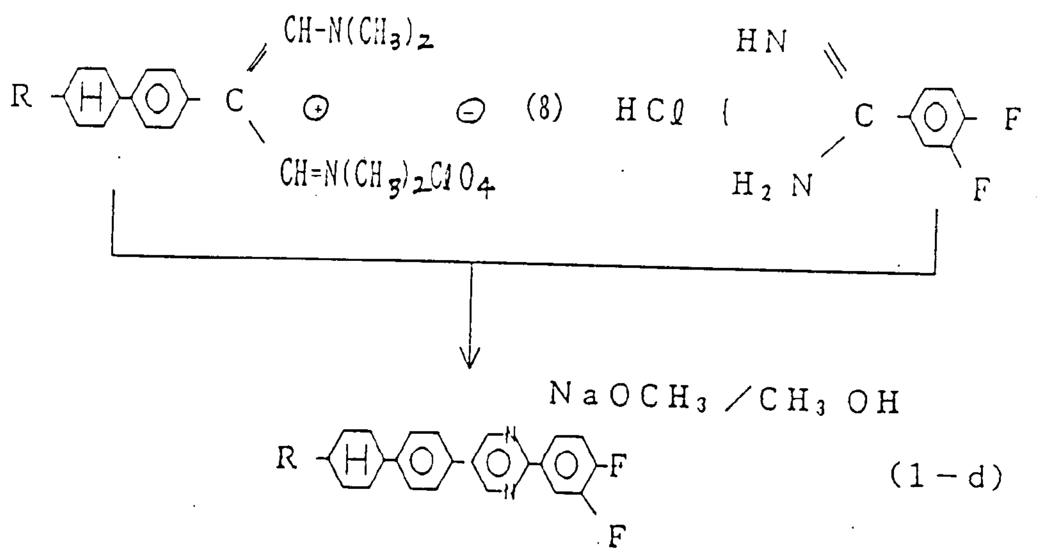


(8) (34)

(1 - d)

where R represents a straight chain alkyl group with 1 to 10 carbon atoms and the cyclohexane ring has a trans arrangement.

**Step 1**

Compound (8) and compound (34) are brought into reaction using sodium methoxide in anhydrous methanol, to obtain 2-(3′,4′-difluorophenyl)-5-(4′-(trans-4″-alkylcyclohexyl)phenyl) pyrimidine (1-e) according to the present invention.

In the present invention, a pyrimidine derivative represented by the general formula:

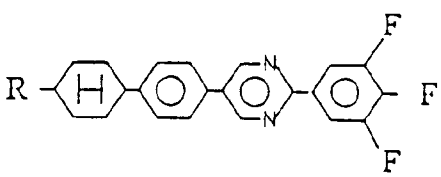

( 1 - f ) :

where R represents a straight chain alkyl group with 1 to 10 carbon atoms and the cyclohexane ring has a trans arrangement, can be produced in accordance with Reaction Schemes 1, 12 and 13. In Reaction Scheme 12, starting from 3,4,5-trifluorobenzonitrile, 3,4,5-trifluorobenzamidine hydrogen chloride (compound (37) is obtained by two reaction steps. In Reaction Scheme 13, starting from compound (8) and compound (37), 2-(3′,4′,5′-trifluorophenyl)-5-(4′-(trans-4″-alkylcyclohexyl)phenyl) pyrimidine (1-f) is obtained.

The outline of the process illustrated in Reaction Scheme 12 is as follows:

Reaction Scheme 12

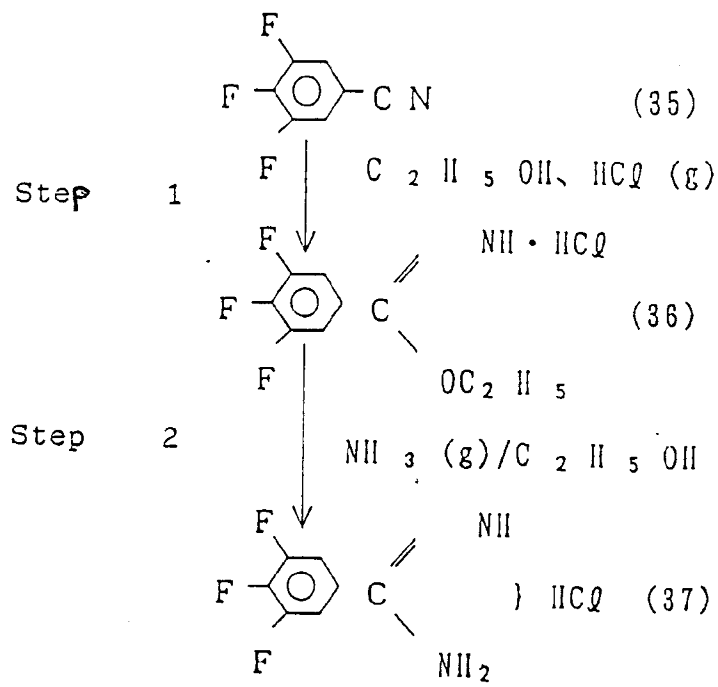


**Step 1**

3,4,5-trifluorobenzonitrile (compound (35)) is brought into reaction with gaseous hydrogen chloride in anhydrous ethanol, to obtain 3,4,5-trifluorobenzimidate hydrogen chloride (compound (36)).

**Step 2**

Compound (36) is reacted with a gaseous ammonia in ethanol to obtain 3,4,5-trifluorobenzamidine hydrogen chloride (compound (37)).

Then in accordance with Reaction Scheme 13:

Reaction Scheme 13

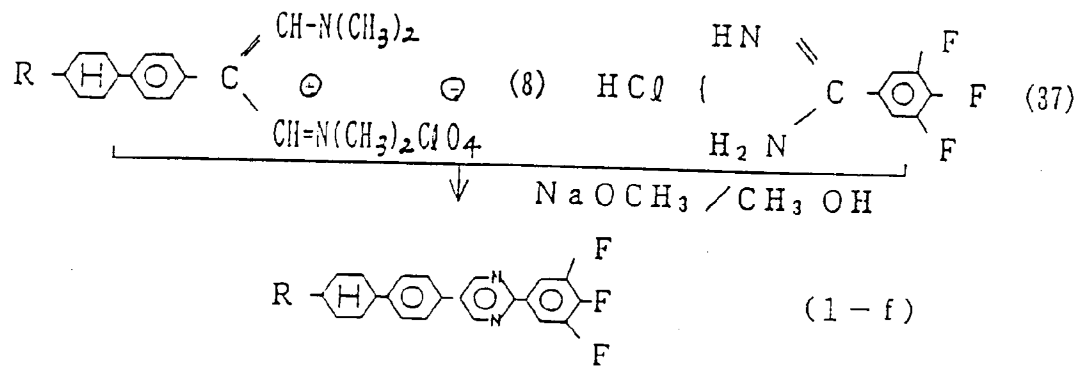


where R represents a straight chain alkyl group with 1 to 10 carbon atoms and the cyclohexane ring has a trans arrangement.

Compound (8) and compound (37) are reacted using sodium methoxide in anhydrous methanol to obtain 2-(3',4',5'-trifluorophenyl)-5-(4'-(trans-4''-alkylcyclohexyl)phenyl) pyrimidine (1-f) according to the present invention.

The present invention will be explained more specifically with reference to the following Examples.

EXAMPLE 1

Process for producing 1-dimethylamino-3-dimethylimino-2-(4'-(trans-4''-propylcyclohexyl)phenyl)-1-propene perchlorate (Reaction Scheme 1).

**Step 1**

128 g (0.52 mol) of 4-(trans-4'-propylcyclohexyl) benzoic acid was refluxed in 75 $cm^3$ (1.0 mol) of $SOCl_2$ for three hours. Excess $SOCl_2$ was distilled off under reduced pressure and the residue was subjected to distillation under reduced pressure (150°C/3.0 mmHg), to obtain 133 g (0.50 mol) of 4-(trans-4'-propylcyclohexyl) benzoic acid chloride.

**Step 2**

100 $cm^3$ of toluene was added to 170 $cm^3$ (0.6 mol) of a 70% toluene solution containing $NaAlH_2(OC_2H_4OCH_3)$, to which 133 g (0.50 mol) of 4-(trans-4'-propylcyclohexyl) benzoic acid chloride was added dropwise while stirring at such a rate to cause moderate refluxing of the toluene and then stirred over a warm water bath at 88 to 90°C for five hours. The reaction solution was cooled to room temperature, and 500 $cm^3$ of concentrated hydro-chloric acid diluted to twice volume was added dropwise. The toluene layer was separated and the aqueous layer was extracted three times, each time with 100 $cm^3$ of toluene and the toluene layers were washed with 10% hydrochloric acid and water. Toluene was distilled off under reduced pressure and the residue was subjected to distillation under reduced pressure (150°C/2.5 mmHg), to obtain 114 g (0.49 mol) of 4-(trans-4'-propylcyclo-hexyl)-benzyl alcohol.

**Step 3**

114 g (0.49 mol) of 4-(trans-4'-propylcyclohexyl) benzyl alcohol was dissolved in 107 $cm^3$ (1.05 mol) of $SOCl_2$ and refluxed over a warm water bath for five hours. Excess $SOCl_2$ was distilled off under reduced pressure and then the residue was washed with 10% hydrochloric acid and water. Chloroform was distilled off under reduced pressure and the residue was subjected to distillation under reduced pressure (147°C/3.0 mmHg) to

obtain 114 g (0.45 mol) of 4-(trans-4′-propylcyclohexyl) benzyl chloride.

**Step 4**

114 g (0.45 mol) of 4-(trans-4′-propylcyclohexyl)benzyl chloride, 27 g (0.55 mol) of NaCN and 113 $cm^3$ of DMSO were heated to 140°C by a mantle heater with stirring. The reaction product was cooled to room temperature. 400 $cm^3$ of water was added, which was extracted three times, each time with 100 $cm^3$ of chloroform and the chloroform layers were washed with 10% hydrochloric acid and water. Chloroform was distilled off under reduced pressure and the residue was subjected to distillation under reduced pressure (155°C/2.5 mmHg), to obtain 106 g (0.44 mol) of 4-(trans-4′-propylcyclohexyl) benzyl cyanide.

**Step 5**

106 g (0.44 mol) of 4-(trans-4′-propylcyclohexyl) benzyl cyanide, 40 g of water, 145 g (2.2 mol) of KOH and 440 $cm^3$ of ethanol were refluxed for ten hours using a mantle heater. Ethanol was distilled off under reduced pressure and the residue was dissolved in 300 $cm^3$ of water, which was poured into 300 $cm^3$ of concentrated hydrochloric acid and 300 g of ice. Deposited crystals were filtered and washed with cold water. The crystals were re-crystallised from methanol to obtain 112 g (0.43 mol) of 4-(trans-4′-propylcyclohexyl)phenyl acetic acid.

**Step 6**

A Vilsmeier reagent was prepared by adding 197 g (1.3 mol) of $POCl_3$ dropwise into 147 g (2.2 mol) of DMF cooled by ice with stirring. To this solution, 112 g (0.43 mol) of powdered 4-(trans-4′-propylcyclohexyl) phenyl acetic acid was added little by little. The two reagents were stirred on a warm water bath at 70 to 80°C for three hours. DMF was distilled off under reduced pressure using a vacuum pump, and the residue was poured into 400 g of ice and then dissolved by stirring. A solution containing 96 g (0.8 mol) of $NaClO_4$ dissolved in 150 $cm^3$ of water was added to the above mentioned solution with stirring and then cooled by iced water. Deposited crystal were filtered and washed with iced water. The crystals were re-crystallised from a mixed solvent of methanol and water, to obtain 160 g (0.37 mol) of 1-dimethylamino-3-dimethylimino-2-(4′-trans-4″-propylcyclohexyl)phenyl)-1-propene perchlorate.

EXAMPLE 2

Process for producing 4-bromobenzamidine hydrogen chloride (Reaction Scheme 2).

**Step 1**

101 g (0.5 mol) of 4-bromobenzoic acid was refluxed in 73 $cm^3$ (1.0 mol) of $SOCl_2$ for three hours on a warm water bath. Excess $SOCl_2$ was distilled off under reduced pressure on the warm water bath by using an aspirator, to obtain 108 g (0.49 mol) of 4-bromobenzoic acid chloride.

**Step 2**

While vigorously stirring 330 $cm^3$ (4.9 mol) of concentrated aqueous ammonia on an iced water bath, 108 g (0.49 mol) of 4-bromobenzoic acid chloride was added dropwise for thirty minutes. The resultant crystals were filtered, washed with water and then re-crystallised from acetone, to obtain 88 g (0.44 mol) of 4-bromobenzoic acid amide.

**Step 3**

88 g (0.44 mol) of 4-bromobenzoic acid amide and 320 $cm^3$ (4.4 mol) of $SOCl_2$ were refluxed on a warm water bath for ten hours. Excess $SOCl_2$ was distilled off on a warm water bath under a reduced pressure by using an aspirator and the residue was re-crystallised from methanol, to obtain 67 g (0.37 mol) of 4-bromobenzonitrile.

**Step 4**

67 g (0.37 mol) of 4-bromobenzonitrile was dissolved in 93 $cm^3$ of anhydrous benzene and 73 $cm^3$ of anhydrous ethanol, which was saturated with gaseous hydrogen chloride, dried with concentrated sulphuric acid while stirring over an iced water bath and then left at a temperature lower than 5°C for two days with the reaction vessel tightly sealed. The solvent in the reaction mixture was distilled off under reduced pressure and the residue was re-crystallised from ethanol to obtain 86 g (0.32 mol) of 4-bromobenzimidate hydrogen chloride.

**Step 5**

86 g (0.32 mol) of 4-bromobenzimidate hydrogen chloride was added to 240 $cm^3$ of ethanol saturated with gaseous ammonia and stirred at room temperature overnight. About one half of the volume of the ethanol was distilled off and the residual solution was re-crystallised, to obtain 57 g (0.24 mol) of 4-bromobenzamidine hydrogen chloride.

EXAMPLE 3

Process for producing 2-(4′-cyanophenyl)-5-(4′-(trans-4″-propylcyclohexyl)phenyl) pyrimidine (Reaction Scheme 3).

**Step 1**

6.9 g (0.03 mol) of sodium was dissolved in 100 $cm^3$ of anhydrous methanol, to which 4.3 g (0.01 mol) of 1-dimethylamino-3-dimethylimino-2-(4′-(trans-4″-propylcyclohexyl)phenyl)-1-propene perchlorate and 3.5 g (0.015 mol) of 4-bromobenzamidine hydrogen chloride were added and left on a warm water bath at 60°C for five hours. Methanol in the reaction product was distilled off and the residue was extracted with chloroform with addition of water and then washed with water, and the chloroform was distilled off. The residue was re-crystallised from chloroform, to obtain 3.3 g (0.008 mol) of 2-(4′-bromophenyl)-5-(4′-(trans-4″-propylcyclohexyl)phenyl) pyrimidine.

**Step 2**

3.3 g of 2-(4′-bromophenyl)-5-(4′-(trans-4″-propyl-cyclohexyl)phenyl) pyrimidine and 0.9 g (0.01 mol) of copper (I) cyanide were added to 30 $cm^3$ of NMP and refluxed for two hours using a mantle heater. The reaction solution was cooled to 60°C and added to a solution comprising 4.4 g of $FeCl_3$-$6H_2O$, 1.3 $cm^3$ of concentrated hydrochloric acid and 6 $cm^3$ of water and then left on a warm water bath at 60°C for one hour. Deposited crystals were filtered, washed with water and then treated by silica gel column chromatography by using chloroform as a solvent. The chloroform was distilled off and the residue was re-crystallised from a mixed solvent of acetone and chloroform to obtain 2.2 g (0.006 mol) of 2-(4′-cyanophenyl)-5-(4′-(trans-4″-propylcyclohexyl)phenyl) pyrimidine. The result of measuring the phase transition point of the compound by DSC was as shown below:

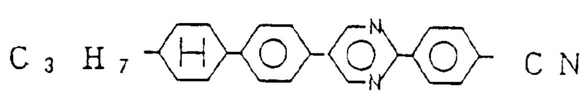

$$C \xrightarrow{194.9^{\circ}C} N \xrightarrow{>360^{\circ}C} I$$

where C represents crystal phase, N represents nematic phase and I represents isotropic liquid phase.

Then, the following compounds were prepared in the same way as those in Examples 1 to 3.

2-(4′-cyanophenyl)-5-(4′-(trans-4″-methylcyclohexyl) phenyl) pyrimidine,

2-(4′-cyanophenyl)-5-(4′-(trans-4″-ethylcyclohexyl) phenyl) pyrimidine,

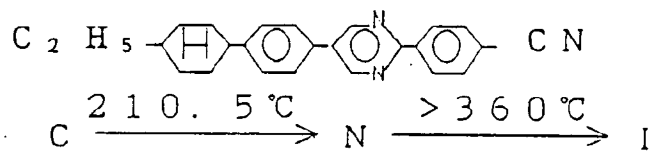


2-(4′-cyanophenyl)-5-(4′-(trans-4″-butylcyclohexyl) phenyl) pyrimidine,

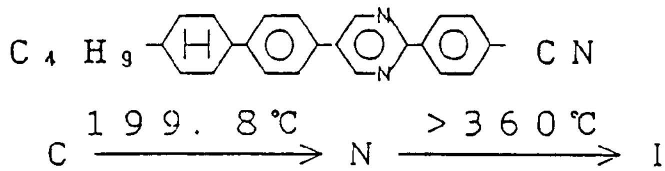


2-(4′-cyanophenyl)-5-(4′-(trans-4″-pentylcyclohexyl) phenyl) pyrimidine,

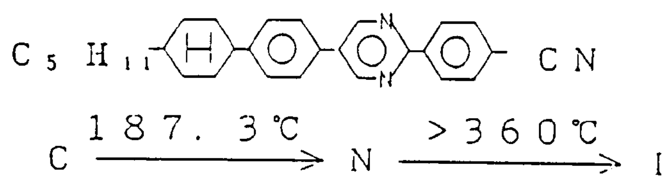


2-(4′-cyanophenyl)-5-(4′-(trans-4″-hexylcyclohexyl) phenyl) pyrimidine,
2- (4′-cyanophenyl)-5-(4′-(trans-4″-heptylcyclohexyl) phenyl) pyrimidine,
2-(4′-cyanophenyl)-5-(4′-(trans-4″-octylcyclohexyl) phenyl) pyrimidine,
2-(4′-cyanophenyl)-5-(4′-(trans-4″-nonylcyclohexyl) phenyl) pyrimidine,
2-(4′-cyanophenyl)-5-(4′-(trans-4″-decylcyclohexyl) phenyl) pyrimidine.

EXAMPLE 4

Process for producing 4-bromo-3-fluorobenzamidine hydrogen chloride (Reaction Scheme 4).

**Step 1**

100 g (0.53 mol) of 4-bromo-fluoroaniline (manufactured by Aldorich Co.) and 70.8 (0.79 mol) of copper (I) cyanide were refluxed while stirring in N-methyl-2-pyrrolidinone for three hours. The reaction solution was cooled, and a solution of 130 cm³ of ethylene diamine and 130 cm³ of water was added dropwise with stirring. The solution was extracted three times, each time with 200 cm³ of chloroform and then washed sufficiently with water. Chloroform was distilled off and the residue was subjected to distillation under reduced pressure (150°C/9 mmHg), to obtain 55 g (0.40 mol) of 4-amino-3-fluorobenzonitrile.

**Step 2**

While stirring 244 cm³ of concentrated sulphuric acid, 30.8 g (0.45 mol) of sodium nitrite was added little by little. After completion of the addition, the solution was heated to 50°C to dissolve any crystals. The solution was added dropwise with iced water cooling and stirring to 406 cm³ of acetic acid. Then, 55 g (0.40 mol) of 4-amino-3-fluorobenzonitrile was added little by little at such a rate as to maintain the temperature less than 25°C and then stirred at 25°C for one hour to dissolve any crystals. Under cooling with iced water and stirring of a solution comprising 87.4 g (0.61 mol) of copper (I) bromide and 244 cm³ of hydrobromic acid (47%), the diazonium salt was added dropwise and then left overnight at room temperature. Deposited crystals were filtered, washed with water and then re-crystallised from hexane to obtain 61.7 g (0.31 mol) of 4-bromo-3-fluorobenzonitrile.

**Step 3**

61.7 g (0.31 mol) of 4-bromo-3-fluoro-benzonitrile was dissolved in 62 $cm^3$ of ethanol and 78 $cm^3$ of benzene and saturated with gaseous hydrogen chloride dried with concentrated sulphuric acid under iced water cooling and, thereafter, left at 5°C for two days. The solvent was distilled off on a warm water bath under reduced pressure using an aspirator. 310 $cm^3$ of ammonia saturated ethanol was added to the residue and stirred overnight at room temperature. Ethanol was distilled off over a warm water bath under reduced pressure. The residue was re-crystallised from ethanol, to obtain 64.7 g (0.26 mol) of 4-bromo-3-fluorobenzamidine hydrogen chloride.

<u>EXAMPLE 5</u>

Process for producing 2-(4′-cyano-3′-fluorophenyl)-5-(4′-(trans-4″-propylcyclohexyl)phenyl) pyrimidine (Reaction Scheme 5).

6.0 g (0.03 mol) of sodium was dissolved in 100 $cm^3$ of methanol to which 4.3 g (0.01 mol) of 1-dimethylamino-3-dimethylimino-2-(4′-(trans-4″-propylcyclohexyl)phenyl)-1-propene perchlorate and 3.1 g (0.012 mol) of 4-bromo-benzamidine hydrogen chloride were added, and left on a warm water bath at 60°C for five hours. Methanol was distilled off and the residue was extracted with chlorform with addition of water and then washed with water and the chloroform was distilled off. Water was added to the residue and crystals were filtered and washed with water. The crystals were re-crystallised from chloroform, to obtain 3.3 g (0.008 mol) of 2-(4′-bromo-3′-fluorophenyl)-5-(4′-(trans-4″-propylcyclohexyl)phenyl) pyrimidine.

**Step 2**

3.5 g (0.008 mol) of 2-(4′-bromo-3′-fluorophenyl)-5-(4′-(trans-4″-propylcyclohexyl)phenyl) pyrimidine and 1.1 g (0.012 mol) of copper (I) cyanide were added to 31 $cm^3$ of NMP and refluxed for two hours using a mantle heater. The reaction solution was cooled to 60°C and added to a solution comprising 4.2 g of $FeCl_3$-$6H_2O$, 1.3 $cm^3$ of concentrated hydrochloric acid and 6 $cm^3$ of water and then left on a warm water bath at 60°C for one hour. Deposited crystals were filtered, washed with water and then treated by silica gel column chromatography using chloroform as a solvent. Chlorofrom was distilled off and the residue was re-crystallised from acetone to obtain 1.9 g (0.005 mol) of 2-(4′-cyano-3′-fluorophenyl)-5-(4′-(trans-4″-propylcyclohexyl) phenyl) pyrimidine. The result of measuring the phase transition point of the compound by DSC was as shown below:

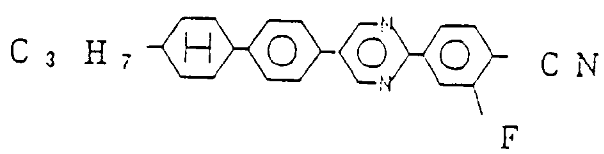

$$C \xrightarrow{157.0℃} N \xrightarrow{362.7℃} I$$

where C represents crystal phase, N represents nematic phase and I represents isotropic liquid phase.

Then, the following compounds were prepared by the same procedures as those in Examples 4 and 5.

2-(4′-cyano-3′-fluorophenyl)-5-(4′-(trans-4″-methylcyclohexyl)phenyl) pyrimidine,

2-(4′-cyano-3′-fluorophenyl)-5-(4′-(trans-4″-ethylcyclohexyl)phenyl) pyrimidine,

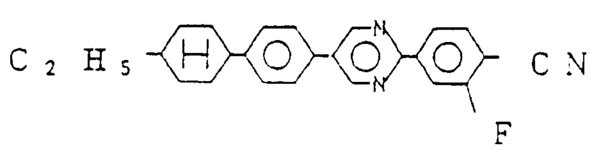

$$C \xrightarrow{173.0℃} N \xrightarrow{349.9℃} I$$

2-(4′-cyano-3′-fluorophenyl)-5-(4′-(trans-4″-butylcyclohexyl)phenyl) pyrimidine,

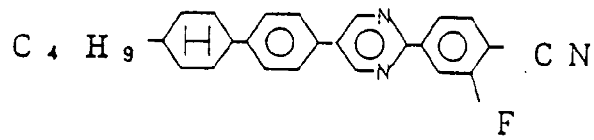

$C \xrightarrow{159.2\ ^\circ C} N \xrightarrow{353.6\ ^\circ C} I$

2-(4′-cyano-3′-fluorophenyl)-5-(4′-(trans-4″-pentylcyclohexyl)phenyl) pyrimidine,

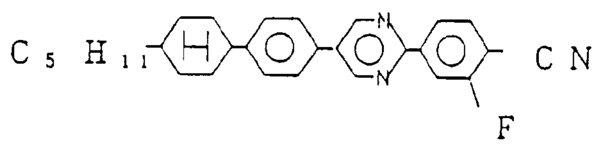

$C \xrightarrow{138.6\ ^\circ C} S \xrightarrow{145.2^\circ C} N \xrightarrow{346.5\ ^\circ C} I$

2-(4′-cyano-3′-fluorophenyl)-5-(4′-(trans-4″-hexylcyclohexyl)phenyl) pyrimidine,
2-(4′-cyano-3′-fluorophenyl)-5-(4′-(trans-4″-heptylcyclohexyl)phenyl) pyrimidine,
2-(4′-cyano-3′-fluorophenyl)-5-(4′-(trans-4″-octylcyclohexyl)phenyl) pyrimidine,
2-(4′-cyano-3′-fluorophenyl)-5-(4′-(trans-4″-nonylcyclohexyl)phenyl) pyrimidine,
2-(4′-cyano-3′-fluorophenyl)-5-(4′-(trans-4″-decylcyclohexyl)phenyl) pyrimidine,

EXAMPLE 6

Process for producing 4-bromo-3,4-difluorobenzamidine hydrogen chloride (Reaction Scheme 6).

**Step 1**

While stirring a solution containing 252 g (1.95 mol) of 2,6-difluoroaniline and 170 g (2.15 mol) of anhydrous pyrimidine dissolved in 780 cm$^3$ of chloroform on an iced water bath, a solution containing 328 g (2.05 mol) of bromine dissolved in 390 cm$^3$ of chloroform was added dropwise for two hours. Then, after stirring at room temperature for two hours, the solution was washed with water. Chloroform was distilled off and the residue was subjected to distillation under reduced pressure (110°C/30 mmHg). The crystals were re-crystallised from hexane, to obtain 340 g (1.63 mol) of 4-bromo-2,6-difluoroaniline.

**Step 2**

340 g (1.63 mol) of 4-bromo-2,6-difluoroaniline and 223 g (2.5 mol) of copper (I) cyanide were refluxed in 820 cm$^3$ of NMP for two hours using a mantle heater. The reaction solution was cooled to room temperature, after adding 300 cm$^3$ of EDA, poured into 2000 cm$^3$ of water, extracted with hexane and then washed with water. Hexane was distilled off and the residue was distillated under reduced pressure (143°C/6 mmHg), to obtain 108 g (0.70 mol) of 4-amino-5-difluorobenzonitrile.

**Step 3**

While stirring 420 cm$^3$ of concentrated sulphuric acid on an iced water bath, 54 g (0.78 mol) of finely powdered $NaNO_2$ was added at such a rate as to maintain the temperature below 40°C and stirred on a warm water bath at 50°C until crystals were completely dissolved. While stirring the solution on an iced water bath, 700 cm$^3$ of glacial acetic acid was added dropwise. Then, 108 g (0.70 mol) of 4-amino-3,5-difluoro-benzonitrile was added at such a rate as to maintain the temperature at 20 to 25°C and stirred at that temperature until the crystals were completely dissolved to prepare a diazonium salt. An aqueous solution of the diazonium salt was added dropwise for two hours to a solution containing 143 g (1.0 mol) of copper (I) bromide dissolved in

420 $cm^3$ of 47% hydrobromic acid while stirring the solution on an iced water bath and then they were stirred for one hour on the iced bath overnight at room temperature. The reaction product was filtered, washed with glacial acetic acid and re-crystallised from a mixed solvent of acetone and methanol, to obtain 98 g (0.45 mol) of 4-bromo-3,5-difluoro-benzonitrile.

**Step 4**

98 g (0.45 mol) of 4-bromo-3,5-difluoro-benzonitrile was dissolved in a mixed solvent of 190 $cm^3$ of anhydrous ethanol and 600 $cm^3$ of anhydrous benzene, to which gaseous hydrogen chloride dried with concentrated sulphuric acid was absorbed to saturation, on an iced water bath. Then, the reaction vessel was tightly sealed and left at a temperature below 5°C for two days. The solvent was distilled off and the residue was washed with ether, to obtain 4-bromo-3,5-difluoro-benzimidate hydrogen chloride.

**Step 5**

450 $cm^3$ of anhydrous ethanol saturated with dried gaseous ammonia was added to 4-bromo-3,5-difluoro-benzimidate hydrogen chloride and stirred overnight at room temperature. About one half amount of the solvent was distilled off and the residue was re-crystallised, to obtain 90 g (0.36 mol) of 4-bromo-3,5-difluoro-benzamidine hydrogen chloride.

<u>EXAMPLE 7</u>

Process for producing 2-(4'-cyano-3',5'-difluorophenyl)-5-(4'(trans-4''-propylcyclohexyl)phenyl) pyrimidine (Reaction Scheme 7).

**Step 1**

0.69 g (0.03 mol) of sodium was dissolved in 100 $cm^3$ of methanol, to which 4.3 g (0.01 mol) of 1-dimethylamino-3-dimethylimino-2-(4'-(trans-4''-propylcyclohexyl)phenyl)-1-propene perchlorate and 4.1 g (0.015 mol) of 4-bromo-benzamidine hydrogen chloride were added and left on a warm water bath at 60°C for five hours. Methanol in the reaction solution was distilled off and the residue was filtered with addition of water and then washed with water. The crystals were re-crystallised from a mixed solvent of acetone and chloroform, to obtain 3.0 g (0.006 mol) of 2-(4'-bromo-3',5'-difluorophenyl)-5-(4'-(trans-4''-propylcyclohexyl)phenyl) pyrimidine.

**Step 2**

3.0 g (0.006 mol) of 2-(4'-bromo-3',5'-difluorophenyl)-5-(4'-(trans-4''-propylcyclohexyl)-phenyl) pyrimidine and 0.8 g (0.08 mol) of copper (I) cyanide were added to 30 $cm^3$ of NMP and refluxed for two hours using a mantle heater. The reaction solution was cooled to 60°C and added to a solution comprising 3.2 g of $FeCl_3$-$6H_2O$, 1.0 $cm^3$ of concentrated hydrochloric acid and 4 $cm^3$ of water and then left on a warm water bath at 60°C for one hour. Resultant crystals were filtered, washed with water and then treated by silica gel column chromatography using chloroform as a solvent. Chloroform was distilled off and the residue was re-crystallised from a mixed solvent of acetone and chloroform, to obtain 1.3 g (0.003 mol) of 2-(4'-cyano-3',5'-difluorophenyl)-5-(4'-(trans-4''-propylcyclohexyl) phenyl) pyrimidine. The result of measuring the phase transition point of the compound by DSC was as shown below:

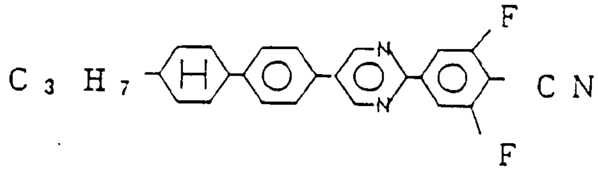

$$C \xrightarrow{154.2^\circ C} N \xrightarrow{320.1^\circ C} I$$

Then, the following compounds were prepared in the same precedures as those in Examples 6 and 7.
2-(4'-cyano-3',5'-difluorophenyl)-5-(4'-(trans-4"-methylcyclohexyl)phenyl) pyrimidine,
2-(4'-cyano-3',5'-difluorophenyl)-5-(4'-(trans-4"-ethylcyclohexyl)phenyl) pyrimidine,

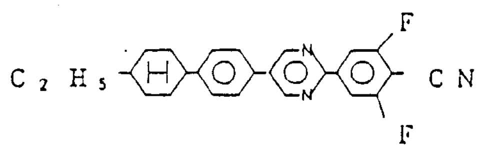

$$C \xrightarrow{154.2^\circ C} N \xrightarrow{304.8^\circ C} I$$

2-(4'-cyano-3',5'-difluorophenyl)-5-(4'-(trans-4"-butylcyclohexyl)phenyl) pyrimidine,

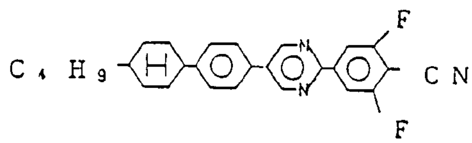

$$C \xrightarrow{144.5^\circ C} N \xrightarrow{312.2^\circ C} I$$

2-(4'-cyano-3',5'-difluorophenyl)-5-(4'-(trans-4"-pentylcyclohexyl)phenyl) pyrimidine,

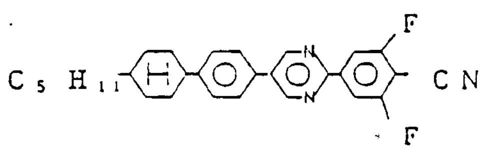

$$C \xrightarrow{150.3^\circ C} N \xrightarrow{307.7^\circ C} I$$

2-(4'-cyano-3',5'-difluorophenyl)-5-(4'-(trans-4"-hexylcyclohexyl)phenyl) pyrimidine,
2-(4'-cyano-3',5'-difluorophenyl)-5-(4'-(trans-4"-heptylcyclohexyl)phenyl) pyrimidine,
2-(4'-cyano-3',5'-difluorophenyl)-5-(4'-(trans-4"-octylcyclohexyl)phenyl) pyrimidine,
2-(4'-cyano-3',5'-difluorophenyl)-5-(4'-(trans-4"-nonylcyclohexyl)phenyl) pyrimidine,
2-(4'-cyano-3',5'-difluorophenyl)-5-(4'-(trans-4"-decylcyclohexyl)phenyl) pyrimidine.

## EXAMPLE 8

Process for producing 4-fluorobenzamidine hydrochloride (Reaction Scheme 8).

**Step 1**

50 g (0.41 mol) of 4-fluorobenzonitrile was dissolved in 82 $cm^3$ of anhydrous ethanol and 103 $cm^3$ of benzene and, under ice cooling, saturated with hydrogen gas and left at a temperature below 5°C for one day and one night. The solvent was distilled off and the residue was crystallised from 100 $cm^3$ of ethanol, to obtain 83 g (0.41 mol) of 4-fluorobenzimidate hydrogen chloride.

**Step 2**

310 $cm^3$ of ethanol saturated with ammonia was added to 83 g (0.41 mol) of 4-fluorobenzimidate hydrogen chloride and stirred at room temperature for one day and one night. About one half the amount of ethanol was distilled off and the residual solution was re-crystallised, to obtain 70 g (0.40 mol) of 4-fluorobenzamidine hydrogen chloride.

EXAMPLE 9

Process for producing 2-(4'-fluorophenyl)-5-(4'-(trans-4"-propylcyclohexyl)phenyl) pyrimidine (Reaction Scheme 9).

0.7 g (0.03 mol) of sodium was dissolved in 100 $cm^3$ of methanol, to which 4.3 g (0.01 mol) of 1-dimethylamino-3-dimethylimino-2-(4'-(trans-4"-propylcyclohexyl)phenyl)-2-propene perchlorate and 2.6 g (0.15 mol) of 4-fluorobenzamidine hydrogen chloride were added and left at 60°C for five hours. The reaction product was cooled to -20°C, crystals were filtered and washed with water. The crystals were treated by silica gel column chromatography using chloroform as a solvent and re-crystallised from a mixed solvent of chloroform and acetone, to obtain 3.2 g (0.009 mol) of 2-(4'-fluorophenyl)-5-(4'-(trans-4"-propylcyclohexyl)phenyl) pyrimidine. The result of measuring the phase transition point of the compound by DSC was as shown below:

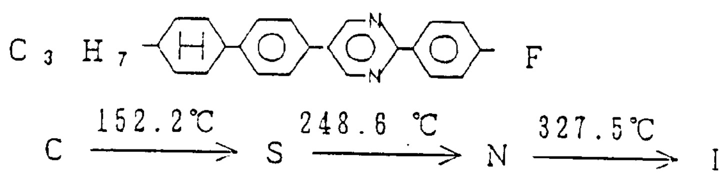


where C represents crystal, N represents nematic phase and I represents isotropic liquid phase.

Then, the following compounds were prepared by the same procedures.

2-(4'-fluorophenyl)-5-(4'-(trans-4"-methylcyclohexyl)phenyl) pyrimidine,
2-(4'-fluorophenyl)-5-(4'-(trans-4"-ethylcyclohexyl)phenyl) pyrimidine,

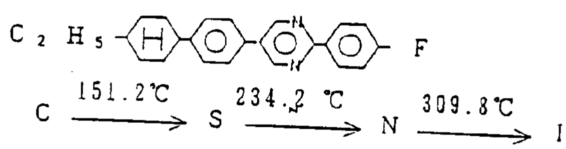


2-(4'-fluorophenyl)-5-(4'-(trans-4"-butylcyclohexyl)phenyl) pyrimidine,

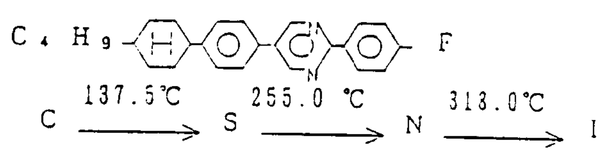


2-(4'-fluorophenyl)-5-(4'-(trans-4"-pentylcyclohexyl)phenyl) pyrimidine,

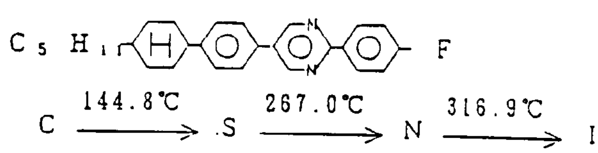


2-(4'-fluorophenyl)-5-(4'-(trans-4"-hexylcyclohexyl)phenyl) pyrimidine,
2-(4'-fluorophenyl)-5-(4'-(trans-4"-heptylcyclohexyl)phenyl) pyrimidine,
2-(4'-fluorophenyl)-5-(4'-(trans-4"-octylcyclohexyl)phenyl) pyrimidine,
2-(4'-fluorophenyl)-5-(4'-(trans-4"-nonylcyclohexyl)phenyl) pyrimidine,
2-(4'-fluorophenyl)-5-(4'-(trans-4"-decylcyclohexyl)phenyl) pyrimidine.

EXAMPLE 10

Process for producing 3,4-difluoro-benzamidine hydrogen chloride (Reaction Scheme 10).

**Step 1**

50 g (0.36 mol) of 3,4-difluoro-benzonitrile (manufactured by Aldorich Co.) was dissolved in 72 cm³ of ethanol and 90 cm³ of benzene and, under cooling with iced water, saturated with HCl gas dried with concentrated $H_2SO_4$ and the reaction vessel was tightly sealed and left at a temperature below 5°C for two days. The solvent in the reaction product was distilled off under reduced pressure over a warm water bath at a temperature below 50°C and the residue was re-crystallised from 80 cm³ of methanol, to obtain 80 g (0.36 mol) of 4-difluoro-benzimidate hydrogen chloride.

**Step 2**

250 cm³ of ethanol was cooled with iced water and ammonia gas was supplied from a gas cylinder to prepare an ammonia saturated ethanol solution. The solution was added to 80 g (0.36 mol) of 4-difluoro-benzimidate hydrogen chloride and stirred at room temperature overnight. Deposited $NH_4Cl$ was filtered and about one half the volume of ethanol in the filtrate was distilled off on a warm water bath and the residue was re-crystallised, to obtain 68 g (0.35 mol) of 3,4-difluoro-benzamidine hydrogen chloride.

EXAMPLE 11

Process for producing 2-(3′,4′-difluorophenyl)-5-(4′-(trans-4″-propylcyclohexyl)phenyl) pyrimidine (Reaction Scheme 11).

0.7 g (0.03 mol of sodium was dissolved in 100 cm³ of methanol, to which 4.3 g (0.01 mol) of 1-dimethylamino-3-dimethylimino-1-(4′-(trans-4″-propylcyclohexyl)phenyl)-2-propene perchlorate and 3.9 g (0.015 mol) of 3,4-bromo-benzamidine hydrogen chloride were added and left on a warm water bath at 60°C for five hours. The reaction product was cooled to -20°C and crystals were filtered and washed with water. The crystals were re-crystallised from a mixed solvent of chloroform and acetone, to obtain 3.3 g (0.0085 mol) of 2-(3′,4′-difluorophenyl)-5-(4′-(trans-4″-propylcyclohexyl)phenyl) pyrimidine. The result of measuring the phase transition point of the compound by DSC was as shown below:

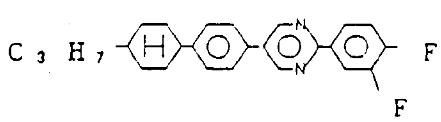

$$C \xrightarrow{143.3^\circ C} S \xrightarrow{202.1^\circ C} N \xrightarrow{294.3^\circ C} I$$

where C represents crystal phase, N represents nematic phase and I represents isotropic liquid phase.

By the same production process, the following compounds were prepared.

2-(3′,4′-difluorophenyl)-5-(4′-(trans-4″-methylcyclohexyl)phenyl) pyrimidine,

2-(3′,4′-difluorophenyl)-5-(4′-(trans-4″-ethylcyclohexyl)phenyl) pyrimidine,

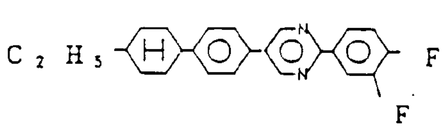

$$C \xrightarrow{144.5^\circ C} S \xrightarrow{190.7^\circ C} N \xrightarrow{271.7^\circ C} I$$

2-(3′,4′-difluorophenyl)-5-(4′-(trans-4″-butylcyclohexyl)phenyl) pyrimidine,

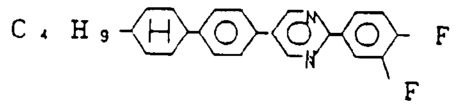

$$C \xrightarrow{102.1\ ^{\circ}C} S_1 \xrightarrow{132.8\ ^{\circ}C} S_2 \xrightarrow{210.6\ ^{\circ}C} N \xrightarrow{286.7\ ^{\circ}C} I$$

where $S_1$ and $S_2$ represent smectic phase respectively.
2-(3′,4′-difluorophenyl)-5-(4′-(trans-4″-pentylcyclohexyl)phenyl) pyrimidine,

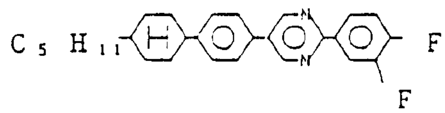

$$C \xrightarrow{114.5\ ^{\circ}C} S_1 \xrightarrow{127.5\ ^{\circ}C} S_2 \xrightarrow{227.5\ ^{\circ}C} N \xrightarrow{287.7\ ^{\circ}C} I$$

2-(3′,4′-difluorophenyl)-5-(4′-(trans-4″-hexylcyclohexyl)phenyl) pyrimidine,
2-(3′,4′-difluorophenyl)-5-(4′-(trans-4″-heptylcyclohexyl)phenyl) pyrimidine,
2-(3′,4′-difluorophenyl)-5-(4′-(trans-4″-octylcyclohexyl)phenyl) pyrimidine,
2-(3′,4′-difluorophenyl)-5-(4′-(trans-4″-nonylcyclohexyl)phenyl) pyrimidine,
2-(3′,4′-difluorophenyl)-5-(4′-(trans-4″-decylcyclohexyl)phenyl) pyrimidine.

EXAMPLE 12

The following compounds were prepared by the same process as those in Examples 10 and 11.
2-(3′,4′,5′-trifluorophenyl)-5-(4′-(trans-4″-methylcyclohexyl)phenyl) pyrimidine,
2-(3′,4′,5′-trifluorophenyl)-5-(4′-(trans-4″-ethylcyclohexyl)phenyl) pyrimidine,
2-(3′,4′,5′-trifluorophenyl)-5-(4′-(trans-4″-propylcyclohexyl)phenyl) pyrimidine,
2-(3′,4′,5′-trifluorophenyl)-5-(4′-(trans-4″-butylcyclohexyl)phenyl) pyrimidine,
2-(3′,4′,5′-trifluorophenyl)-5-(4′-(trans-4″-pentylcyclohexyl)phenyl) pyrimidine,
2-(3′,4′,5′-trifluorophenyl)-5-(4′-(trans-4″-hexylcyclohexyl)phenyl) pyrimidine,
2-(3′,4′,5′-trifluorophenyl)-5-(4′-(trans-4″-heptylcyclohexyl)phenyl) pyrimidine,
2-(3′,4′,5′-trifluorophenyl)-5-(4′-(trans-4″-octylcyclohexyl)phenyl) pyrimidine,
2-(3′,4′,5′-trifluorophenyl)-5-(4′-(trans-4″-nonylcyclohexyl)phenyl) pyrimidine,
2-(3′,4′,5′-trifluorophenyl)-5-(4′-(trans-4″-decylcyclohexyl)phenyl) pyrimidine.

EXAMPLE 13

A compound according to the present invention in which R represents

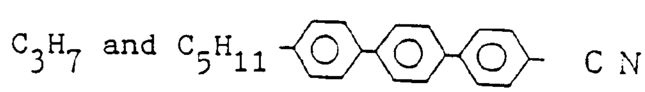

and

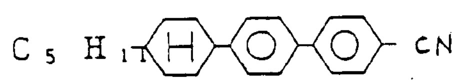

as comparative examples were mixed each in an amount of 10% by weight with 90% by weight of a commercially available nematic liquid crystal composition ZLI-1565 (manufactured by Merk Co.), to prepare a liquid crystal composition. The N-I point of these liquid crystal compositions and threshold voltage (Vth) for voltage luminance characteristics when sealing the composition in a TN type liquid crystal display cell of 9 μm thickness were measured and the results are shown in Table 2.

Table 2

| Compound | (N-I)point(°C) | Vth (V) |
|---|---|---|
| ZLI-1565 90<br>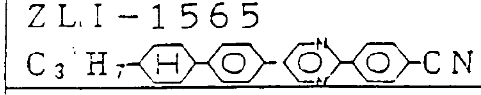 10 | 111 | 2.49 |
| ZLI-1565 90<br>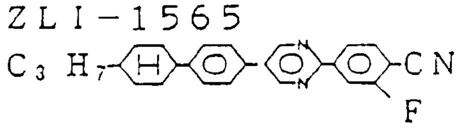 10 | 108 | 2.37 |
| ZLI-1565 90<br>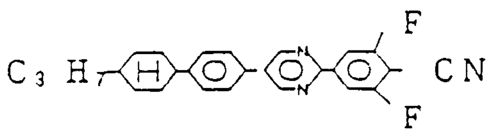 10 | 104 | 2.12 |
| ZLI-1565 90<br>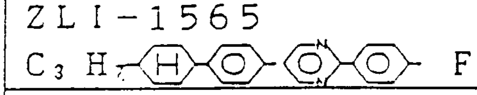 10 | 105 | 2.56 |
| ZLI-1565 90<br>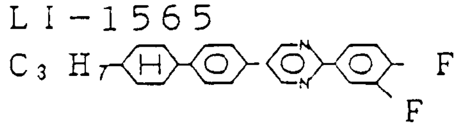 10 | 103 | 2.46 |
| ZLI-1565 90<br>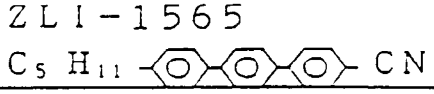 10 | 101 | 2.51 |
| ZLI-1565 90<br>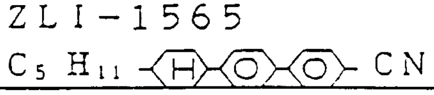 10 | 99 | 2.47 |
| ZLI-1565 100 | 87 | 2.43 |

As has been described above, the pyrimidine derivative according to the present invention has an extremely high N-I point (higher than 280°C). Further, it has been confirmed that a liquid crystal composition having a higher N-I point and low threshold voltage for voltage luminance characteristics can be obtained by mixing a pyrimidine compound according to the present invention with a conventional liquid crystal composition.

Accordingly, the pyrimidine compounds according to the present invention are extremely useful compounds used for TN type or STN type liquid crystal display devices.

## Claims

1. A pyrimidine derivative characterised by the general formula:

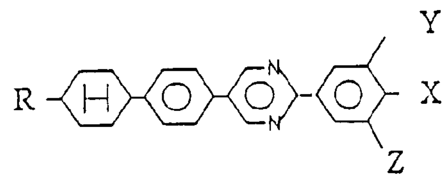

where R represents a straight chain alkyl group with 1 to 10 carbon atoms, X represents F or CN, Y and Z represent, respectively, H or F and the cyclohexane ring has a trans arrangement.

2. A pyrimidine derivative as claimed in claim 1, characterised by the general formula:

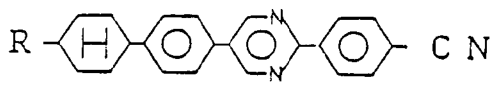 (1 − a)

where R represents a straight chain alkyl group with 1 to 10 carbon atoms and the cyclohexane ring has a trans arrangement.

3. A pyrimidine derivative as claimed in claim 1, characterised by the general formula:

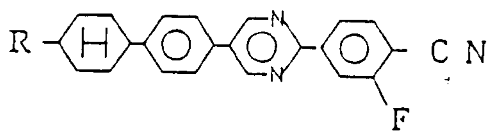 (1 − b)

where R represents a straight chain alkyl group with 1 to 10 carbon atoms and the cyclohexane ring has a trans arrangement.

4. A pyrimidine derivative as claimed in claim 1, characterised by the general formula:

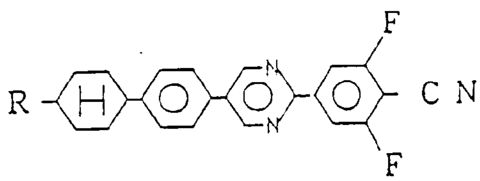 (1 − c)

where R represents a straight chain alkyl group with 1 to 10 carbon atoms and the cyclohexane ring has a trans arrangement.

5. A pyrimidine derivative as claimed in claim 1, characterised by the general formula:

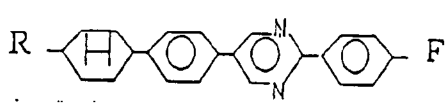 (1 − d)

where R represents a straight chain alkyl group with 1 to 10 carbon atoms and the cyclohexane ring has a trans arrangement.

6. A pyrimidine derivative as claimed in claim 1, characterised by the general formula:

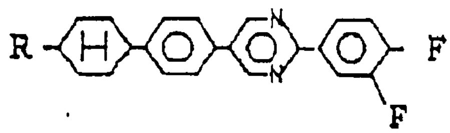

(1 - e)

where R represents a straight chain alkyl group with 1 to 10 carbon atoms and the cyclohexane ring has a trans arrangement.

7. A pyrimidine derivative as claimed in claim 1, characterised by the general formula:

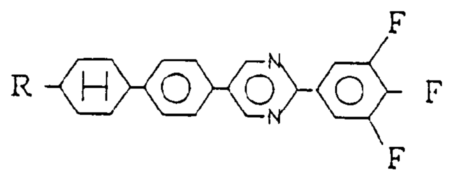

(1 - f)

where R represents a straight chain alkyl group with 1 to 10 carbon atoms and the cyclohexane ring has a trans arrangement.

8. 2-(4′-cyanophenyl)-5-(4′(trans-4″-propylcyclohexyl)-phenyl) pyrimidine, or
2-(4′-cyanophenyl)-5-(4′-(trans-4″-methylcyclohexyl) phenyl) pyrimidine, or
2-(4′-cyanophenyl)-5-(4′-(trans-4″-ethylcyclohexyl) phenyl) pyrimidine, or
2-(4′-cyanophenyl)-5-(4′-(trans-4″-butylcyclohexyl) phenyl) pyrimidine, or
2-(4′-cyanophenyl)-5-(4′-(trans-4″-pentylcyclohexyl) phenyl) pyrimidine, or
2-(4′-cyanophenyl)-5-(4′-(trans-4″-hexylcyclohexyl) phenyl) pyrimidine, or
2-(4′-cyanophenyl)-5-(4′-(trans-4″-heptylcyclohexyl) phenyl) pyrimidine, or
2-(4′-cyanophenyl)-5-(4′-(trans-4″-octylcyclohexyl) phenyl) pyrimidine, or
2-(4′-cyanophenyl)-5-(4′-(trans-4″-nonylcyclohexyl) phenyl) pyrimidine, or
2-(4′-cyanophenyl)-5-(4′-(trans-4″-decylcyclohexyl) phenyl) pyrimidine, or
2-(4′-cyano-3′-fluorophenyl)-5-(4′-(trans-4″-propylcyclohexyl)-phenyl) pyrimidine, or
2-(4′-cyano-3′-fluorophenyl)-5-(4′-(trans-4″-methylcyclohexyl)phenyl) pyrimidine, or
2-(4′-cyano-3′-fluorophenyl)-5-(4′-(trans-4″-ethylcyclohexyl)phenyl) pyrimidine, or
2-(4′-cyano-3′-fluorophenyl)-5-(4′-(trans-4″-butylcyclohexyl)phenyl) pyrimidine, or
2-(4′-cyano-3′-fluorophenyl)-5-(4′-(trans-4″-pentylcyclohexyl)phenyl) pyrimidine, or
2-(4′-cyano-3′-fluorophenyl)-5-(4′-(trans-4″-hexylcyclohexyl)phenyl) pyrimidine, or
2-(4′-cyano-3′-fluorophenyl)-5-(4′-(trans-4″-heptylcyclohexyl)phenyl) pyrimidine, or
2-(4′-cyano-3′-fluorophenyl)-5-(4′-(trans-4″-octylcyclohexyl)phenyl) pyrimidine, or
2-(4′-cyano-3′-fluorophenyl)-5-(4′-(trans-4″-nonylcyclohexyl)phenyl) pyrimidine, or
2-(4′-cyano-3′-fluorophenyl)-5-(4′-(trans-4″-decylcyclohexyl)phenyl) pyrimidine, or
2-(4′-cyano-3′,5′-difluorophenyl)-5-(4′-(trans-4″-propylcyclohexyl)phenyl) pyrimidine, or
2-(4′-cyano-3′,5′-difluorophenyl)-5-(4′-(trans-4″-methylcyclohexyl)phenyl) pyrimidine, or
2-(4′-cyano-3′,5′-difluorophenyl)-5-(4′-(trans-4″-ethylcyclohexyl)phenyl) pyrimidine, or
2-(4′-cyano-3′,5′-difluorophenyl)-5-(4′-(trans-4″-butylcyclohexyl)phenyl) pyrimidine, or
2-(4′-cyano-3′,5′-difluorophenyl)-5-(4′-(trans-4″-pentylcyclohexyl)phenyl) pyrimidine, or
2-(4′-cyano-3′,5′-difluorophenyl)-5-(4′-(trans-4″-hexylcyclohexyl)phenyl) pyrimidine, or
2-(4′-cyano-3′,5′-difluorophenyl)-5-(4′-(trans-4″-heptylcyclohexyl)phenyl) pyrimidine, or
2-(4′-cyano-3′,5′-difluorophenyl)-5-(4′-(trans-4″-octylcyclohexyl)phenyl) pyrimidine, or
2-(4′-cyano-3′,5′-difluorophenyl)-5-(4′-(trans-4″-nonylcyclohexyl)phenyl) pyrimidine, or
2-(4′-cyano-3′,5′-difluorophenyl)-5-(4′-(trans-4″-decylcyclohexyl)phenyl) pyrimidine, or
2-(4′-fluorophenyl)-5-(4-(trans-4″-propylcyclohexyl)phenyl) pyrimidine, or
2-(4′-fluorophenyl)-5-(4′-(trans-4″-methylcyclohexyl)phenyl) pyrimidine, or
2-(4′-fluorophenyl)-5-(4′-(trans-4″-ethylcyclohexyl)phenyl) pyrimidine, or
2-(4′-fluorophenyl)-5-(4′-(trans-4″-butylcyclohexyl)phenyl) pyrimidine, or
2-(4′-fluorophenyl)-5-(4′-(trans-4″-pentylcyclohexyl)phenyl) pyrimidine, or
2-(4′-fluorophenyl)-5-(4′-(trans-4″-hexylcyclohexyl)phenyl) pyrimidine, or
2-(4′-fluorophenyl)-5-(4′-(trans-4″-heptylcyclohexyl)phenyl) pyrimidine, or

2-(4′-fluorophenyl)-5-(4′-(trans-4″-octylcyclohexyl)phenyl) pyrimidine, or
2-(4′-fluorophenyl)-5-(4′-(trans-4″-nonylcyclohexyl)phenyl) pyrimidine, or
2-(4′-fluorophenyl)-5-(4′-(trans-4″-decylcyclohexyl)phenyl) pyrimidine, or
2-(3′,4′-difluorophenyl)-5-(4′-(trans-4″-propylcyclohexyl)-phenyl) pyrimidine, or
2-(3′,4′-difluorophenyl)-5-(4′-(trans-4″-methylcyclohexyl)phenyl) pyrimidine, or
2-(3′,4′-difluorophenyl)-5-(4′-(trans-4″-ethylcyclohexyl)phenyl) pyrimidine, or
2-(3′,4′-difluorophenyl)-5-(4′-(trans-4″-butylcyclohexyl)phenyl) pyrimidine, or
2-(3′,4′-difluorophenyl)-5-(4′-(trans-4″-pentylcyclohexyl)phenyl) pyrimidine, or
2-(3′,4′-difluorophenyl)-5-(4′-(trans-4″-hexylcyclohexyl)phenyl) pyrimidine, or
2-(3′,4′-difluorophenyl)-5-(4′-(trans-4″-heptylcyclohexyl)phenyl) pyrimidine, or
2-(3′,4′-difluorophenyl)-5-(4′-(trans-4″-octylcyclohexyl)phenyl) pyrimidine, or
2-(3′,4′-difluorophenyl)-5-(4′-(trans-4″-nonylcyclohexyl)phenyl) pyrimidine, or
2-(3′,4′-difluorophenyl)-5-(4′-(trans-4″-decylcyclohexyl)phenyl) pyrimidine, or
2-(3′,4′,5′-trifluorophenyl)-5-(4′-(trans-4″-methylcyclohexyl)phenyl) pyrimidine, or
2-(3′,4′,5′-trifluorophenyl)-5-(4′-(trans-4″-ethylcyclohexyl)phenyl) pyrimidine, or
2-(3′,4′,5′-trifluorophenyl)-5-(4′-(trans-4″-propylcyclohexyl)phenyl) pyrimidine, or
2-(3′,4′,5′-trifluorophenyl)-5-(4′-(trans-4″-butylcyclohexyl)phenyl) pyrimidine, or
2-(3′,4′,5′-trifluorophenyl)-5-(4′-(trans-4″-pentylcyclohexyl)phenyl) pyrimidine, or
2-(3′,4′,5′-trifluorophenyl)-5-(4′-(trans-4″-hexylcyclohexyl)phenyl) pyrimidine, or
2-(3′,4′,5′-trifluorophenyl)-5-(4′-(trans-4″-heptylcyclohexyl)phenyl) pyrimidine, or
2-(3′,4′,5′-trifluorophenyl)-5-(4′-(trans-4″-octylcyclohexyl)phenyl) pyrimidine, or
2-(3′,4′,5′-trifluorophenyl)-5-(4′-(trans-4″-nonylcyclohexyl)phenyl) pyrimidine, or
2-(3′,4′,5′-trifluorophenyl)-5-(4′-(trans-4″-decylcyclohexyl)phenyl) pyrimidine.

9. A liquid crystal composition, characterised by comprising at least one pyrimidine derivative as claimed in any preceding claim.

10. A liquid crystal composition as claimed in claim 8, characterised by comprising from 3 to 20% by weight of one or more pyrimidine derivatives as claimed in any of claims 1 to 8.

**Patentansprüche**

1. Pyrimidin-Derivat,
**gekennzeichnet durch**
die allgemeine Formel:

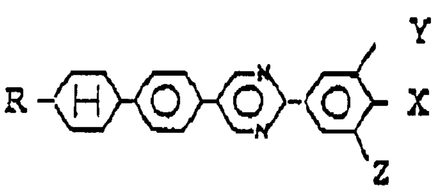

in der R eine geradkettige Alkyl-Gruppe mit 1 bis 10 Kohlenstoffatomen darstellt, X F oder CN darstellt, Y und Z jeweils H oder F darstellen, und der Cyclohexan-Ring eine trans-Anordnung hat.

2. Pyrimidin-Derivat nach Anspruch 1,
**gekennzeichnet durch**
die allgemeine Formel:

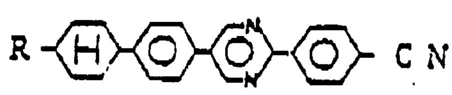

(1 - a)

in der R eine geradkettige Alkyl-Gruppe mit 1 bis 10 Kohlenstoffatomen darstellt und der Cyclohexan-Ring eine trans-Anordnung hat.

3. Pyrimidin-Derivat nach Anspruch 1, **gekennzeichnet durch** die allgemeine Formel:

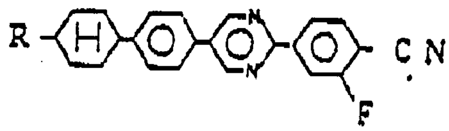

(I-b)

in der R eine geradkettige Alkyl-Gruppe mit 1 bis 10 Kohlenstoffatomen darstellt und der Cyclohexan-Ring eine trans-Anordnung hat.

4. Pyrimidin-Derivat nach Anspruch 1, **gekennzeichnet durch** die allgemeine Formel:

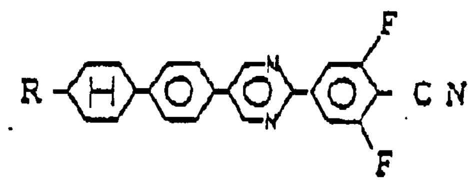

(I-c)

in der R eine geradkettige Alkyl-Gruppe mit 1 bis 10 Kohlenstoffatomen darstellt und der Cyclohexan-Ring eine trans-Anordnung hat.

5. Pyrimidin-Derivat nach Anspruch 1, **gekennzeichnet durch** die allgemeine Formel:

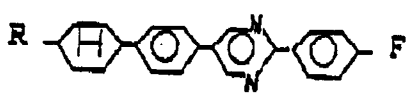

(I-d)

in der R eine geradkettige Alkyl-Gruppe mit 1 bis 10 Kohlenstoffatomen darstellt und der Cyclohexan-Ring eine trans-Anordnung hat.

6. Pyrimidin-Derivat nach Anspruch 1, **gekennzeichnet durch** die allgemeine Formel:

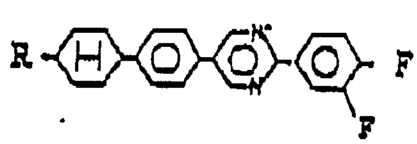

(I-e)

in der R eine geradkettige Alkyl-Gruppe mit 1 bis 10 Kohlenstoffatomen darstellt und der Cyclohexan-Ring eine trans-Anordnung hat.

7. Pyrimidin-Derivat nach Anspruch 1, **gekennzeichnet durch** die allgemeine Formel:

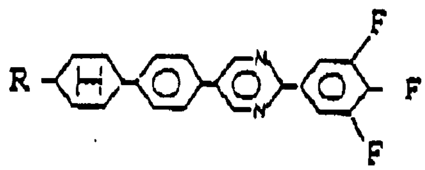

(1 - f)

in der R eine geradkettige Alkyl-Gruppe mit 1 bis 10 Kohlenstoffatomen darstellt und der Cyclohexan-Ring eine trans-Anordnung hat.

8. 2-(4′-Cyanophenyl)-5-(4′(trans-4″-propylcyclohexyl)-phenyl) pyrimidin, oder
2-(4′-Cyanophenyl)-5-(4′(trans-4″-methylcyclohexyl )-phenyl) pyrimidin, oder
2-(4′-Cyanophenyl)-5-(4′(trans-4″-ethylcyclo hexyl)-phenyl) pyrimidin, oder
2-(4′-Cyanophenyl)-5-(4′(trans-4″-butylcyclohexyl)-phenyl) pyrimidin, oder
2-(4′-Cyanophenyl)-5-(4′(trans-4″-pentylcyclohexyl)phenyl) pyrimidin, oder
2-(4′-Cyanophenyl)-5-(4′(trans-4″-hexylcyclohexyl)phenyl) pyrimidin, oder
2-(4′-Cyanophenyl)-5-(4′(trans-4″-heptylcylcohexyl)phenyl) pyrimidin, oder
2-(4′-Cyanophenyl)-5-(4′(trans-4″-octylcyclohexyl)phenyl) pyrimidin, oder
2-(4′-Cyanophenyl)-5-(4′(trans-4″-nonylcyclohexyl)phenyl) pyrimidin, oder
2-(4′-Cyanophenyl)-5-(4′(trans-4″-decylcyclohexyl)phenyl) pyrimidin, oder
2-(4′-Cyano-3′-fluorphenyl)-5-(4′(trans-4″-propylcyclohexyl)phenyl) pyrimidin, oder
2-(4′-Cyano-3′-fluorphenyl)-5-(4’(trans-4″-methylcyclohexyl)phenyl) pyrimidin, oder
2-(4′-Cyano-3′-fluorphenyl)-5-(4′(trans-4″-ethylcyclohexyl)phenyl) pyrimidin, oder
2-(4′-Cyano-3′-fluorphenyl)-5-(4′(trans-4″-butylcyclohexyl)phenyl) pyrimidin, oder
2-(4′-Cyano-3′-fluorphenyl)-5-(4′-(trans-4″-pentylcyclohexyl)phenyl) pyrimidin, oder
2-(4′-Cyano-3′-fluorphenyl)-5-(4′(trans-4″-hexylcyclohexyl)phenyl) pyrimidin, oder
2-(4′-Cyano-3′-fluorphenyl)-5-(4′(trans-4″-heptylcyclohexyl)phenyl) pyrimidin, oder
2-(4′-Cyano-3′-fluorphenyl)-5-(4′(trans-4″-octylcyclohexyl)phenyl) pyrimidin, oder
2-(4′-Cyano-3′-fluorphenyl)-5-(4′(trans-4″-nonylcylcohexyl)phenyl) pyrimidin, oder
2-(4′-Cyano-3′-fluorphenyl)-5-(4′(trans-4″-decylcyclohexyl)phenyl) pyrimidin, oder
2-(4′-Cyano-3′,5′-difluorphenyl)-5-(4′(trans-4″-propylcyclohexyl)phenyl pyrimidin, oder
2-(4′-Cyano-3′,5-difluorphenyl)-5-(4′(trans-4″-methylcyclohexyl)phenyl) pyrimidin, oder
2-(4′-Cyano-3′,5′-difluorphenyl)-5-(4′(trans-4″-ethylcyclohexyl)phenyl) pyrimidin, oder
2-(4′-Cyano-3′,5′-difluorphenyl)-5-(4′(trans-4″-butylcyclohexyl)phenyl) pyrimidin, oder
2-(4′-Cyano-3′,5′-difluorphenyl)-5-(4′(trans-4″-pentylcylcohexyl)phenyl) pyrimidin, oder
2-(4′-Cyano-3′,5′-difluorphenyl)-5-(4′(trans-4″-hexylcyclohexyl)phenyl) pyrimidin, oder
2-(4′-Cyano-3′,5′-difluorphenyl)-5-(4′(trans-4″-heptylcyclohexyl)phenyl) pyrimidin, oder
2-(4′-Cyano-3′,5′-difluorphenyl)-5-(4′(trans-4″-octylcyclohexyl)phenyl) pyrimidin, oder
2-(4′-Cyano-3′,5′-difluorphenyl)-5-(4′(trans-4″-nonylcylcohexyl)phenyl) pyrimidin, oder
2-(4′-Cyano-3′,5′-difluorphenyl)-5-(4′(trans-4″-decylcyclohexyl)phenyl) pyrimidin, oder
2-(4′-Fluorphenyl)-5-(4′-(trans-4″-propylcyclohexyl)phenyl)pyrimidin, oder
2-(4′-Fluorphenyl)-5-(4′-(trans-4″-methylcyclohexyl)phenyl)pyrimidin, oder
2-(4′-Fluorphenyl)-5-(4′-(trans-4″-ethylcyclohexyl)phenyl)pyrimidin, oder
2-(4′-Fluorphenyl)-5-(4′-(trans-4″-butylcyclohexyl)phenyl)pyrimidin, oder
2-(4′-Fluorphenyl)-5-(4′-(trans-4″-pentylcyclohexyl)phenyl)pyrimidin, oder
2-(4′-Fluorphenyl)-5-(4′-(trans-4″-hexylcyclohexyl)phenyl)pyrimidin, oder
2-(4′-Fluorphenyl)-5-(4′-(trans-4″-heptycyclohexyl)phenyl)pyrimidin, oder
2-(4′-Fluorphenyl)-5-(4′-(trans-4″-octylcyclohexyl)phenyl)pyrimidin, oder
2-(4′-Fluorphenyl)-5-(4′-(trans-4″-nonylcyclohexyl)phenyl)pyrimidin, oder
2-(4′-Fluorphenyl)-5-(4′-(trans-4″-decylcyclohexyl)phenyl)pyrimidin, oder
2-(3′,4′-Difluorphenyl)-5-(4′-trans-4″-propylcyclohexyl)phenyl) pyrimidin, oder
2-(3′,4′-Difluorphenyl)-5-(4′-trans-4″-methylcyclohexyl)phenyl) pyrimidin, oder
2-(3′,4′-Difluorphenyl)-5-(4′-trans-4″-ethylcyclohexyl)phenyl) pyrimidin, oder
2-(3′,4′-Difluorphenyl)-5-(4′-trans-4″-butylcyclohexyl)phenyl) pyrimidin, oder
2-(3′,4′-Difluorphenyl)-5-(4′-trans-4″-pentylcyclohexyl)phenyl) pyrimidin, oder
2-(3′,4′-Difluorphenyl)-5-(4′-trans-4″-hexylcyclohexyl)phenyl) pyrimidin, oder
2-(3′,4′-Difluorphenyl)-5-(4′-trans-4″-heptylcyclohexyl)phenyl) pyrimidin, oder
2-(3′,4′-Difluorphenyl)-5-(4′-trans-4″-octylcyclohexyl)phenyl) pyrimidin, oder

2-(3′,4′-Difluorphenyl)-5-(4′-trans-4″-nonylcyclohexyl)phenyl) pyrimidin, oder
2-(3′,4′-Difluorphenyl)-5-(4′-trans-4″-decylcyclohexyl)phenyl) pyrimidin, oder
2-(3′,4′,5′-Trifluorphenyl)-5-(4′-(trans-4″-methylcyclohexyl)phenyl) pyrimidin, oder
2-(3′,4′,5′-Trifluorphenyl)-5-(4′-(trans-4″-ethylcyclohexyl)phenyl) pyrimidin, oder
2-(3′,4′,5′-Trifluorphenyl)-5-(4′-(trans-4″-propylcyclohexyl)phenyl) pyrimidin, oder
2-(3′,4′,5′-Trifluorphenyl)-5-(4′-(trans-4″-butylcyclohexyl)phenyl) pyrimidin, oder
2-(3′,4′,5′-Trifluorphenyl)-5-(4′-(trans-4″-pentylcyclohexyl)phenyl) pyrimidin, oder
2-(3′,4′,5′-Trifluorphenyl)-5-(4′-(trans-4″-hexylcyclohexyl)phenyl) pyrimidin, oder
2-(3′,4′,5′-Trifluorphenyl)-5-(4′-(trans-4″-heptylcyclohexyl)phenyl) pyrimidin, oder
2-(3′,4′,5′-Trifluorphenyl)-5-(4′-(trans-4″-octylcyclohexyl)phenyl) pyridimin, oder
2-(3′,4′,5′-Trifluorphenyl)-5-(4′-(trans-4″-nonylcyclohexyl)phenyl) pyrimidin, oder
2-(3′,4′,5′-Trifluorphenyl)-5-(4′-(trans-4″-decylcyclohexyl)phenyl) pyrimidin.

9. Flüssigkristall-Zusammensetzung,
**dadurch gekennzeichnet,**
daß sie mindestens ein Pyrimidin-Derivat wie in irgendeinem vorhergehenden Anspruch beansprucht, aufweist.

10. Flüssigkristall-Zusammensetzung nach Anspruch 9,
**dadurch gekennzeichnet,**
daß sie 3 bis 20 Gew.-% eines Pyrimidin-Derivats oder mehrerer Pyrimidin-Derivate wie in irgendeinem der Ansprüche 1 bis 8 beansprucht, aufweist.

## Revendications

1. Dérivé de la pyrimidine caractérisé par la formule générale :

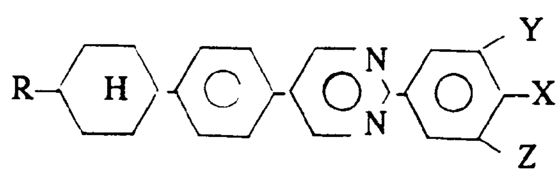

où R représente un groupe alkyle à chaîne droite avec 1 à 10 atomes de carbone, X représente F ou CN, Y et Z représentent respectivement H ou F et le groupe cyclohexane a une configuration trans.

2. Dérivé de la pyrimidine selon la revendication 1, caractérisé par la formule générale :

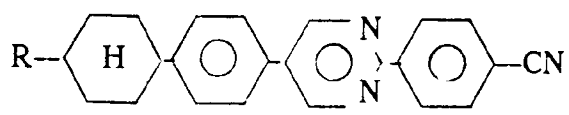

(1 - a)

où R représente un groupe alkyle à chaîne droite avec 1 à 10 atomes de carbone, et le groupe cyclohexane a une configuration trans.

3. Dérivé de la pyrimidine selon la revendication 1, caractérisé par la formule générale :

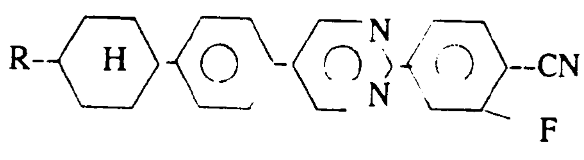

(1 - b)

où R représente un groupe alkyle à chaîne droite avec 1 à 10 atomes de carbone, et le groupe cyclohexane a une configuration trans.

4. Dérivé de la pyrimidine selon la revendication 1, caractérisé par la formule générale :

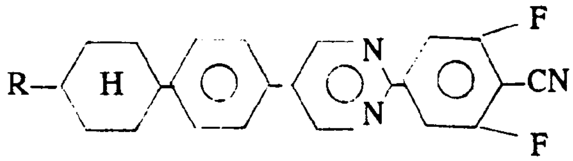

(1 - c)

où R représente un groupe alkyle à chaîne droite avec 1 à 10 atomes de carbone, et le groupe cyclohexane a une configuration trans.

5. Dérivé de la pyrimidine selon la revendication 1, caractérisé par la formule générale :

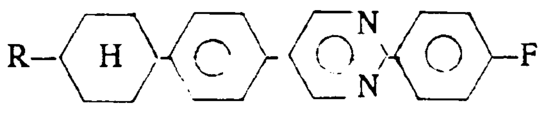

(1 - d)

où R représente un groupe alkyle à chaîne droite avec 1 à 10 atomes de carbone, et le groupe cyclohexane a une configuration trans.

6. Dérivé de la pyrimidine selon la revendication 1, caractérisé par la formule générale :

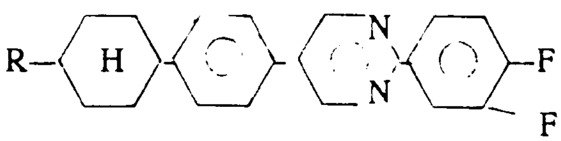

(1 - e)

où R représente un groupe alkyle à chaîne droite avec 1 à 10 atomes de carbone, et le groupe cyclohexane a une configuration trans.

7. Dérivé de la pyrimidine selon la revendication 1, caractérisé par la formule générale :

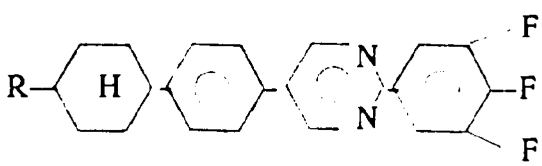

(1 - f)

où R représente un groupe alkyle à chaîne droite avec i à 10 atomes de carbone, et le groupe cyclohexane a une configuration trans.

8. 2-(4′-cyanophényl)-5-(4′-(trans-4″-propylcyclohexyl)-phényl) pyrimidine, ou
2-(4′-cyanophényl)-5-(4′-(trans-4″-méthylcyclohexyl)-phényl) pyrimidine, ou
2-(4′-cyanophényl)-5-(4′-(trans-4″-éthylcyclohexyl)-phényl) pyrimidine, ou
2-(4′-cyanophényl)-5-(4′-(trans-4″-butylcyclohexyl)-phényl) pyrimidine, ou
2-(4′-cyanophényl)-5-(4′-(trans-4″-pentylcyclohexyl)-phényl) pyrimidine, ou
2-(4′-cyanophényl)-5-(4′-(trans-4″-hexylcyclohexyl)-phényl) pyrimidine, ou
2-(4′-cyanophényl)-5-(4′-(trans-4″-heptylcyclohexyl)-phényl) pyrimidine, ou
2-(4′-cyanophényl)-5-(4′-(trans-4″-octylcyclohexyl)-phényl) pyrimidine, ou
2-(4′-cyanophényl)-5-(4′-(trans-4″-nonylcyclohexyl)-phényl) pyrimidine, ou
2-(4′-cyanophényl)-5-(4′-(trans-4″-décylcyclohexyl)-phényl) pyrimidine, ou
2-(4′-cyano-3′-fluorophényl)-5-(4′-(trans-4″-propylcyclohexyl)-phényl) pyrimidine, ou
2-(4′-cyano-3′-fluorophényl)-5-(4′-(trans-4″-méthylcyclohexyl)-phényl) pyrimidine, ou
2-(4′-cyano-3′-fluorophényl)-5-(4′-(trans-4″-éthylcyclohexyl)-phényl) pyrimidine, ou

2-(4′-cyano-3′-fluorophényl)-5-(4′-(trans-4″-butylcyclohexyl)-phényl) pyrimidine, ou
2-(4′-cyano-3′-fluorophényl)-5-(4′-(trans-4″-pentylcyclohexyl)-phényl) pyrimidine, ou
2-(4′-cyano-3′-fluorophényl)-5-(4′-(trans-4″-hexylcyclohexyl)-phényl) pyrimidine, ou
2-(4′-cyano-3′-fluorophényl)-5-(4 ′-(trans-4″-heptylcyclohexyl)-phényl) pyrimidine, ou
2-(4′-cyano-3′-fluorophényl)-5-(4′-(trans-4″-octylcyclohexyl)-phényl) pyrimidine, ou
2-(4′-cyano-3′-fluorophényl)-5-(4′-(trans-4″-nonylcyclohexyl)-phényl) pyrimidine, ou
2-(4′-cyano-3′-fluorophényl)-5-(4′-(trans-4″-décylcyclohexyl)-phényl) pyrimidine, ou
2-(4′-cyano-3′,5′-difluorophényl)-5-(4′-(trans-4″-propylcyclohexyl)-phényl) pyrimidine, ou
2-(4′-cyano-3′,5′-difluorophényl)-5-(4′-(trans-4″-méthylcyclohexyl)-phényl) pyrimidine, ou
2-(4′-cyano-3′,5′-difluorophényl)-5-(4′-(trans-4″-éthylcyclohexyl)-phényl) pyrimidine, ou
2-(4′-cyano-3′,5′-difluorophényl)-5-(4′-(trans-4″-butylcyclohexyl)-phényl) pyrimidine, ou
2-(4′-cyano-3′,5′-difluorophényl)-5-(4′-(trans-4″-pentylcyclohexyl)-phényl) pyrimidine, ou
2-(4′-cyano-3′,5′-difluorophényl)-5-(4′-(trans-4″-hexylcyclohexyl)-phényl) pyrimidine, ou
2-(4′-cyano-3′,5′-difluorophényl)-5-(4′-(trans-4″-heptylcyclohexyl)-phényl) pyrimidine, ou
2-(4′-cyano-3′,5′-difluorophényl)-5-(4′-(trans-4″-octylcyclohexyl)-phényl) pyrimidine, ou
2-(4′-cyano-3′,5′-difluorophényl)-5-(4′-(trans-4″-nonylcyclohexyl)-phényl) pyrimidine, ou
2-(4′-cyano-3′,5′-difluorophényl)-5-(4′-(trans-4″-décylcyclohexyl)-phényl) pyrimidine, ou
2-(4′-fluorophényl)-5-(4′-(trans-4″-propylcyclohexyl)-phényl) pyrimidine, ou
2-(4′-fluorophényl)-5-(4′-(trans-4″-méthylcyclohexyl)-phényl) pyrimidine, ou
2-(4′-fluorophényl)-5-(4′-(trans-4″-éthylcyclohexyl)-phényl) pyrimidine, ou
2-(4′-fluorophényl)-5-(4′-(trans-4″-butylcyclohexyl)-phényl) pyrimidine, ou
2-(4′-fluorophényl)-5-(4′-(trans-4″-pentylcyclohexyl)-phényl) pyrimidine, ou
2-(4′-fluorophényl)-5-(4′-(trans-4″-hexylcyclohexyl)-phényl) pyrimidine, ou
2-(4′-fluorophényl)-5-(4′-(trans-4″-heptylcyclohexyl)-phényl) pyrimidine, ou
2-(4′-fluorophényl)-5-(4′-(trans-4″-octylcyclohexyl)-phényl) pyrimidine, ou
2-(4′-fluorophényl)-5-(4′-(trans-4″-nonylcyclohexyl)-phényl) pyrimidine, ou
2-(4′-fluorophényl)-5-(4′-(trans-4″-décylcyclohexyl)-phényl) pyrimidine, ou
2-(3′,4′-difluorophényl)-5-(4′-(trans-4″-propylcyclohexyl)-phényl) pyrimidine, ou
2-(3′,4′-difluorophényl)-5-(4′-(trans-4″-méthylcyclohexyl)-phényl) pyrimidine, ou
2-(3′,4′-difluorophényl)-5-(4′-(trans-4″-éthylcyclohexyl)-phényl) pyrimidine, ou
2-(3′,4′-difluorophényl)-5-(4′-(trans-4″-butylcyclohexyl)-phényl) pyrimidine, ou
2-(3′,4′-difluorophényl)-5-(4′-(trans-4″-pentylcyclohexyl)-phényl) pyrimidine, ou
2-(3′,4′-difluorophényl)-5-(4′-(trans-4″-hexylcyclohexyl)-phényl) pyrimidine, ou
2-(3′,4′-difluorophényl)-5-(4′-(trans-4″-heptylcyclohexyl)-phényl) pyrimidine, ou
2-(3′,4′-difluorophényl)-5-(4′-(trans-4″-octylcyclohexyl)-phényl) pyrimidine, ou
2-(3′,4′-difluorophényl)-5-(4′-(trans-4″-nonylcyclohexyl)-phényl) pyrimidine, ou
2-(3′,4′-difluorophényl)-5-(4′-(trans-4″-décylcyclohexyl)-phényl) pyrimidine, ou
2-(3′,4′,5′-trifluorophényl)-5-(4′-(trans-4″-méthylcyclohexyl)-phényl) pyrimidine, ou
2-(3′,4′,5′-trifluorophényl)-5-(4′-(trans-4″-éthylcyclohexyl)-phényl) pyrimidine, ou
2-(3′,4′,5′-trifluorophényl)-5-(4′-(trans-4″-propylcyclohexyl)-phényl) pyrimidine, ou
2-(3′,4′,5′-trifluorophényl)-5-(4′-(trans-4″-butylcyclohexyl)-phényl) pyrimidine, ou
2-(3′,4′,5′-trifluorophényl)-5-(4′-(trans-4″-pentylcyclohexyl)-phényl) pyrimidine, ou
2-(3′,4′,5′-trifluorophényl)-5-(4′-(trans-4″-hexylcyclohexyl)-phényl) pyrimidine, ou
2-(3′,4′,5′-trifluorophényl)-5-(4′-(trans-4″-heptylcyclohexyl)-phényl) pyrimidine, ou
2-(3′,4′,5′-trifluorophényl)-5-(4′-(trans-4″-octylcyclohexyl)-phényl) pyrimidine, ou
2-(3′,4′,5′-trifluorophényl)-5-(4′-(trans-4″-nonylcyclohexyl)-phényl) pyrimidine, ou
2-(3′,4′,5′-trifluorophényl)-5-(4′-(trans-4″-décylcyclohexyl)-phényl) pyrimidine.

**9.** Composition de cristaux liquides caractérisée en ce qu'elle comprend au moins un des dérivés de la pyrimidine revendiqués dans une quelconque des revendications précédentes.

**10.** Composition de cristaux liquides selon la revendication 8, caractérisée en ce qu'elle comprend de 3 à 20% en poids d'un ou de plusieurs dérivés de la pyrimidine revendiqués dans une quelconque des revendications 1 à 8.